# EUROPEAN PATENT SPECIFICATION

(11) **EP 3 618 685 B1**
(45) Date of publication and mention of the grant of the patent: **16.06.2021**
(21) Application number: 18722747.5
(22) Date of filing: 17.04.2018
(51) Int. Cl.: A61B 1/00

(54) **MEDICAL IMAGE PROCESSING APPARATUS, MEDICAL IMAGE PROCESSING METHOD AND ENDOSCOPE SYSTEM**
MEDIZINISCHE BILDVERARBEITUNGSVORRICHTUNG, MEDIZINISCHES BILDVERARBEITUNGSVERFAHREN UND ENDOSKOPSYSTEM
APPAREIL DE TRAITEMENT D'IMAGE MÉDICALE, PROCÉDÉ DE TRAITEMENT D'IMAGE MÉDICALE ET SYSTÈME D'ENDOSCOPE

(30) Priority: 01.05.2017 JP 2017091329
(43) Date of publication of application: 11.03.2020
(73) Proprietor: Sony Corporation, Tokyo 108-0075 (JP)
(72) Inventor: YAMANE Masahito, Tokyo 108-0075 (JP); HIRAYAMA Tomoyuki, Tokyo 108-0075 (JP); SUGIE Yuki, Tokyo 108-0075 (JP); NAKANO Takahito, Tokyo 108-0075 (JP)
(74) Representative: D Young & Co LLP
(86) International application number: PCT/JP2018/015811
(87) International publication number: WO 2018/203473

(56) References cited:
- EP-A1- 3 136 719
- WO-A1-2017/022367
- US-A1- 2013 258 358
- US-A1- 2016 189 417

## Description

### [Technical Field]

The present technology relates to a medical image processing apparatus, a medical image processing method and an endoscope system, and particularly to a medical image processing apparatus, a medical image processing method and an endoscope system by which, for example, lower latency can be implemented.

### [Background Art]

For a medical image that is picked up by an endoscope and is used for medical use, it is demanded to perform processes from image pickup to display in low latency because a doctor performs a medical operation and so forth while watching the medical image.

For example, an image processing apparatus has been proposed in which an image is divided into a plurality of regions lined up in a horizontal direction and a plurality of GPUs (Graphical Processing Units) are used to perform parallel processing for individually processing the plurality of regions to implement low latency (for example, refer to PTL 1).

### [Citation List]

### [Patent Literature]

[PTL 1] WO 2015/163171. Other proposed arrangements are disclosed in EP 3136719, US 2013/258358, WO2017/022367, and US 2016/189417.

### [Summary of Invention]

### [Technical Problem]

In recent years, together with improvement in performance of an image sensor for picking up an image, a medical image picked up by an endoscope or the like is becoming an image of multiple pixels (high pixels) such as a so-called 4K image or 8K image. In order to perform processes from image pickup to display of such a medical image of multiple pixels in low latency, parallel processing using a plurality of GPUs is effective as described in PTL 1.

However, the image processing apparatus described in PTL 1 is configured based on a PC (Personal Computer) architecture, and a medical image before processed or a medical image after processed by a plurality of GPUs is transferred to and stored into a memory managed by a CPU (Central Processing Unit).

Accordingly, even if the speed of processing performed by the GPUs is increased, a period of time required for transfer of a medical image between the GPUs and the memory managed by the CPU is generated at least as latency.

The present technology has been made in view of such a situation as described above and makes it possible to perform processes from pickup to display of a medical image in lower latency.

### Solution to Problem

A surgical endoscope system a surgical endoscope generating medical image data, and an image processing apparatus having switching control circuitry receiving the medical image data generated by the surgical endoscope and performing distribution and aggregation, a plurality of graphic processing circuits performing image processing on the medical image data received via distribution from the switching control circuitry, central processing circuitry connected to the switching circuitry and to the plurality of graphic processing circuits via the switching control circuitry, and memory circuitry managed by the central processing circuitry,
wherein results from the image processing on the image data performed by the plurality of graphic processing circuits are aggregated by the switching control circuitry, and wherein the aggregation of the results is independent of the memory circuitry managed by the central processing circuitry before the results are output to the memory circuitry via the central processing circuitry.

An image processing apparatus including switching control circuitry performing distribution and aggregation, a plurality of graphic processing circuits performing image processing on image data received via distribution from the switching control circuitry, central processing circuitry connected to the switching circuitry and to the plurality of graphic processing circuits via the switching control circuitry, and a memory circuitry managed by the central processing circuitry, wherein results from the image processing on the image data performed by the plurality of graphic processing circuits is aggregated by the switching control circuitry, and wherein the aggregation of the results is performed independent of the memory circuitry managed by the central processing circuitry before the results are output to the memory circuitry via the central processing circuitry.

An image processing method including performing, using a plurality of graphic processing circuits, image processing on medical image data generated by a surgical endoscope or microscope and received via distribution from switching control circuitry, and aggregating, by the switching control circuitry, results from the image processing on the image data performed by the plurality of graphic processing circuits, wherein the aggregation of the results is performed independent of a memory managed by a central processing circuitry before the results are output to the memory via the central processing circuitry, the central processing circuitry connected to the switching circuitry and to the plurality of graphic processing circuits via the switching control circuitry.

### Advantageous Effects of Invention

With the present technology, lower latency can be implemented.

It is to be noted that the effect described here is not necessarily restrictive but may be any one of effects described herein.

### Brief Description of Drawings

[fig. 1] FIG. 1 is a view depicting an example of a configuration of an embodiment of an endoscopic surgery system to which the present technology is applied.
[fig.2]FIG. 2 is a block diagram depicting an example of a CCU 201 which is not an embodiment of the present invention.
[fig.3]FIG. 3 is a view illustrating an outline of image stabilization (process) performed for a medical image by the CCU 201.
[fig.4]FIG. 4 is a flow chart illustrating an outline of an example of image processing including image stabilization performed for a medical image by the CCU 201.
[fig.5]FIG. 5 is a view illustrating that, upon starting of image processing, the efficiency of parallel processing is degraded significantly when image processing in which a necessary region of a medical image is unknown is to be performed.
[fig.6]FIG. 6 is a view illustrating an example of distribution aggregation when image processing including image stabilization is performed for a processing target image.
[fig.7]FIG. 7 is a flow chart illustrating an example of processing of the first configuration example of the CCU 201 when distribution aggregation is performed by a CPU 303.
[fig.8]FIG. 8 is a block diagram depicting a configuration example of the CCU 201 which is an embodiment of the present invention.
[fig.9]FIG. 9 is a block diagram depicting a configuration example of a distribution aggregation unit 312.
[fig.10]FIG. 10 is a block diagram depicting a configuration example of a GPU 315ᵢ.
[fig. 11]FIG. 11 is a flow chart illustrating an example of processing of the second configuration example of the CCU 201.
[fig.12]FIG. 12 is a view illustrating an example of timings of processing of the first configuration example of the CCU 201 and the second configuration example of the CCU 201.
[fig.13]FIG. 13 is a view depicting a first example of a data flow when processing is performed by the second configuration example of the CCU 201.
[fig.14]FIG. 14 is a view depicting a second example of a data flow when processing is performed by the second configuration example of the CCU 201.
[fig.15]FIG. 15 is a view depicting a third example of a data flow when processing is performed by the second configuration example of the CCU 201.
[fig.16]FIG. 16 is a view depicting an example of a processing target image that is made a processing target by the CCU 201.
[fig.17]FIG. 17 is a block diagram depicting another configuration example of the distribution aggregation unit 312.
[fig.18]FIG. 18 is a block diagram depicting a third configuration example of the CCU 201.
[fig.19]FIG. 19 is a block diagram depicting a configuration example of an embodiment of a computer to which the present technology can be applied.

### Description of Embodiments

### <Embodiment of endoscopic surgery system to which present technology is applied>

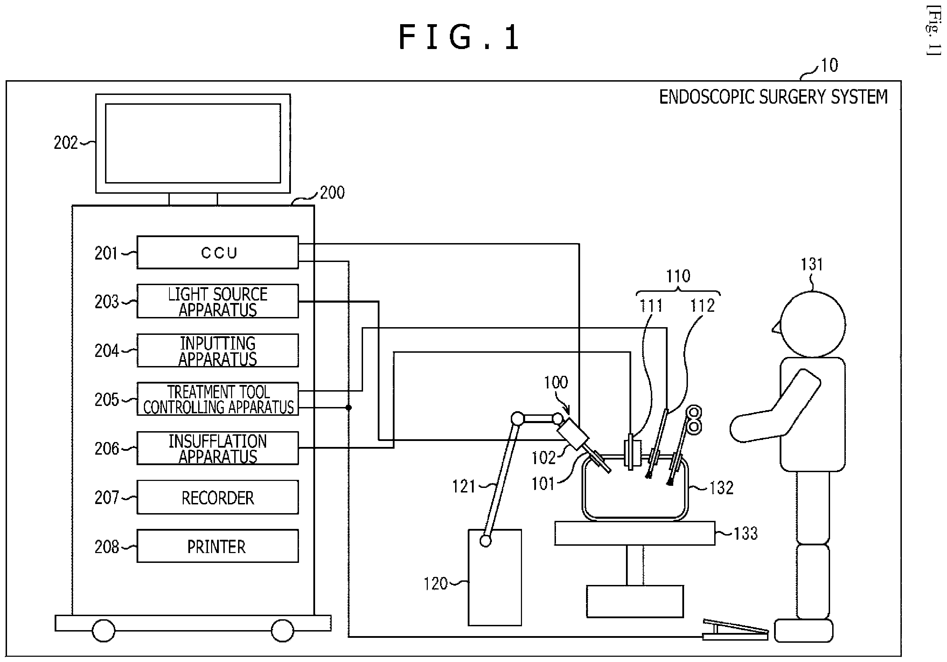

FIG. 1 is a view depicting a configuration example of an embodiment of an endoscopic surgery system to which the present technology is applied.

In FIG. 1, a manner is illustrated in which an operator 131 (doctor) performs surgery for a patient 132 on a patient bed 133 using the endoscopic surgery system 10. As depicted in FIG. 1, the endoscopic surgery system 10 is configured from an endoscope 100, surgical tools 110 such as an insufflation tube 111 and an energy treatment tool 112, a supporting arm apparatus 120 that supports the endoscope 100, and a cart 200 on which various apparatus for endoscopic surgery are carried.

The endoscope 100 is configured from a lens barrel 101 a portion of which having a predetermined length is inserted from a distal end thereof into a lumen of the patient 132, a camera head 102 connected to a proximal end of the lens barrel 101. Although, in the example depicted, the endoscope 100 configured as a so-called rigid mirror having a rigid lens barrel 101 is depicted, the endoscope 100 may otherwise be configured as a so-called flexible mirror having a flexible lens barrel.

At a distal end of the lens barrel 101, an opening in which an objective lens is to be fitted is provided. A light source apparatus 203 is connected to the endoscope 100 such that light generated by the light source apparatus 203 is guided to the distal end of the lens barrel 101 by a light guide extending in the inside of the lens barrel 101 and is irradiated toward an observation target in the lumen of the patient 132 through the objective lens. It is to be noted that the endoscope 100 may be a direct view mirror, a perspective mirror or a side view mirror.

In the inside of the camera head 102, an optical system and an image sensor (image pickup element) are provided such that reflected light (observation light) from an observation target is condensed upon the image sensor by the optical system. The observation light is photoelectrically converted by the image sensor to generate an electric signal corresponding to the observation light, that is, an image signal corresponding to an observation image. The image signal is transmitted as RAW data to a camera control unit (CCU: Camera Control Unit) 201.

It is to be noted that, by generation of an image signal by (an image sensor of) the camera head 102, that is, by pickup of an image, a medical image of multiple pixels such as, for example, a 4K image or an 8K image, can be picked up.

The CCU 201 is configured from a CPU (Central Processing Unit), a GPU (Graphics Processing Unit) or the like and comprehensively controls operation of the endoscope 100 and a display apparatus 202. Further, the CCU 201 receives an image signal (image data) from the camera head 102 and performs various image processes for displaying a medical image corresponding to the image signal, such as, for example, a development process (demosaic process) for the image signal.

In other words, the CCU 201 functions as a medical image processing device that processes a medical image picked up by (the camera head 102 of) the endoscope 100.

The display apparatus 202 displays a medical image corresponding to an image signal, for which image processing has been performed by the CCU 201, under the control by the CCU 201.

The light source apparatus 203 is configured from a light source such as, for example, an LED (Light Emitting Diode) and supplies irradiation light to the endoscope 100 when an image of an observation target such as an operative part is picked up.

An inputting apparatus 204 is an input interface to the endoscopic surgery system 10. A user can perform inputting of various kinds of information, instruction or the like inputting to the endoscopic surgery system 10 through the inputting apparatus 204. For example, the user will input an instruction to change an image pickup condition (type of irradiation light, magnification, focal length or the like) for the endoscope 100.

A treatment tool controlling apparatus 205 controls driving of the energy treatment tool 112 for ablation of a tissue, incision, sealing of a blood vessel or the like. An insufflation apparatus 206 sends gas into a lumen of the patient 132 through the insufflation tube 111 to inflate the lumen in order to secure the field of view by the endoscope 100 and secure a work space for the operator (user). A recorder 207 is an apparatus that can record therein various information relating to surgery. A printer 208 is an apparatus that can print various kinds of information relating to surgery in various forms such as a text, an image, a graph, or the like.

It is to be noted that the light source apparatus 203 that supplies irradiation light when an image of an operative part is to be picked up to the endoscope 100 can be configured from a white light source configured, for example, from an LED, a laser light source or a combination of them. Where the white light source is configured from a combination of RGB (Red, Green and Blue) laser light sources, the output intensity and the output timing for each color (each wavelength) can be controlled with high accuracy. Therefore, the light source apparatus 203 can perform adjustment of the white balance of an image. Further, in this case, by irradiating laser beams from the respective RGB laser light sources time-divisionally upon an operative part and controlling driving of the image sensor of the camera head 102 in synchronism with irradiation timings of the laser beams, it is possible to pick up images corresponding to the respective R, G and B colors time-divisionally. Where such control of driving of the image sensor as just described is performed, a color image can be obtained even if no color filter is provided in the image sensor.

Further, driving of the light source apparatus 203 may be controlled such that the intensity of light to be outputted is changed after every predetermined period of time. By controlling driving of the image sensor of the camera head 102 in synchronism with each timing of a change in intensity of light to acquire images time-divisionally and synthesizing the images, an image of a high dynamic range free from so-called crushed black and overexposure can be produced.

Further, the light source apparatus 203 may be configured such that it can supply light of a predetermined wavelength band suitable for special light observation. In the special light observation, so-called narrow bandwidth light observation (Narrow Band Imaging) is performed by which, for example, utilizing a wavelength dependency of absorption of light by a body issue, light of a narrow band in comparison with irradiation light upon ordinary observation (that is, white light) is irradiated to pick up an image of a predetermined tissue such as a blood vessel of a mucosal surface layer in a high contrast. Alternatively, in the special light observation, fluorescence observation may be performed by which an image is obtained from fluorescent light generated by irradiation of excitation light. In the fluorescence observation, it is possible to observe fluorescent light from the body tissue (autofluorescence observation) by irradiating excitation light upon a body tissue, to obtain a fluorescence image by locally injecting reagent such as indocyanine green (ICG) or the like into a body tissue and then irradiating excitation light corresponding to a fluorescent light wavelength of the reagent upon the body tissue, or the like. The light source apparatus 203 can be configured to be able to supply narrow band light and/or excitation light suitable for such special light observation as described above.

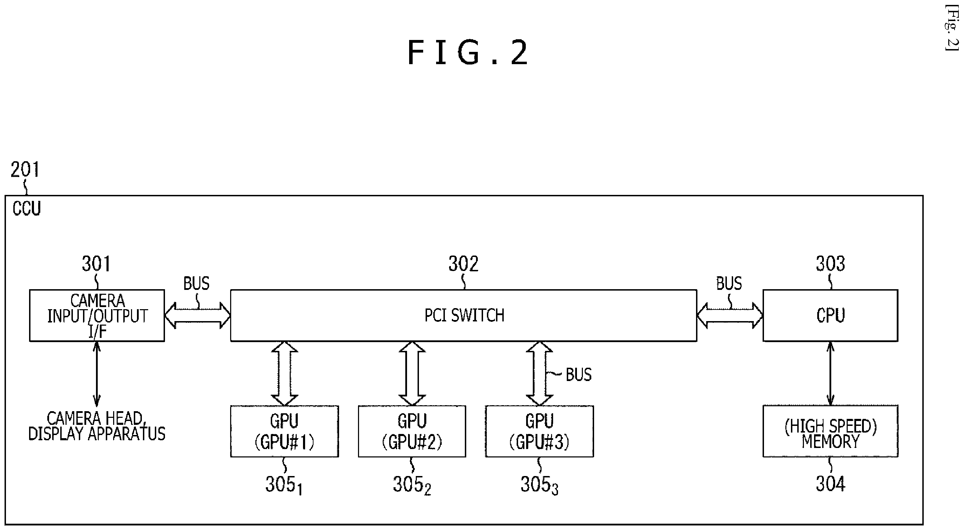

FIG. 2 is a block diagram depicting an example of the CCU 201 of FIG. 1, which is not an embodiment of the present invention.

Referring to FIG. 2, the CCU 201 is configured based on a PC architecture and includes a camera input/output I/F (Interface) 301, a PCI (Peripheral Component Interconnect) switch 302, a CPU (Central Processing Unit (central processing circuitry)) 303 and a memory 304 as well as a plurality of, for example, three, GPUs 305₁, 305₂ and 305₃ (graphic processing circuits). The PCI switch 302 may be one of switching control circuitry, for example.

The camera input/output I/F 301 is an I/F for exchanging a medical image to and from the camera head 102 or the display apparatus 202 of FIG. 1, and supplies (image data of) a medical image picked up by the endoscope 100 and supplied from the camera head 102 to the PCI switch 302 and supplies a medical image supplied from the PCI switch 302 to the display apparatus 202.

The PCI switch 302 is connected to the camera input/output I/F 301, CPU 303 and GPUs 305₁ to 305₃ through a bus. The PCI switch 302 relays exchange of a medical image or other data between the camera input/output I/F 301, CPU 303 and GPUs 305ᵢ in accordance with a bus standard of the PCI.

Accordingly, the camera input/output I/F 301, PCI switch 302, CPU 303 and GPUs 305₁ to 305₃ have a built-in I/F of PCI as a bus I/F for connection to the bus.

The CPU 303 controls the entire CCU 201 in accordance with a predetermined program. For example, the CPU 303 manages the memory 304 (memory circuitry) such that it stores a medical image supplied from the PCI switch 302 into the memory 304 and reads out and supplies a medical image stored in the memory 304 to the PCI switch 302. It is to be noted that a program to be executed by the CPU 303 can be installed in advance into the memory 304, can be installed from a recording medium not depicted into the memory 304, can be downloaded from a site and installed into the memory 304 or the like.

The memory 304 stores medical images and other data under the management of the CPU 303. It is to be noted that, while, in FIG. 2, reading and writing of a medical image from and into the memory 304 are performed, it is desirable to adopt a high speed memory for the memory 304 from a point of view that processes from image pickup to display of a medical image are performed in low latency. Further, reading and writing of a medical image or other data from and into the memory 304 can be performed by DMA (Direct Memory Access). Although reading and writing of data from and into the memory 304 can be performed without involvement of the CPU 303, the memory 304 still is a memory that is managed by the CPU 303.

The GPU 305ᵢ (in FIG. 2, i = 1, 2, 3) is an example of an image processing unit performing image processing for a medical image supplied from the PCI switch 302, and supplies a medical image after image processing to the PCI switch 302.

Although, here in FIG. 2, the three GPUs 305₁ to 305₃ are provided in the CCU 201, the number of GPUs is not limited to three. In particular, a plural number of 2 or 4 or more GPUs can be provided in the CCU 201.

In the CCU 201 configured in such a manner as described above, the camera input/ output I/F 301 outputs a medical image supplied from the camera head 102 to the CPU 303 through the PCI switch 302, and the CPU 303 stores the medical image outputted from the camera input/output I/F 301 into the memory 304.

Further, the CPU 303 reads out all or part of medical images stored in the memory 304 and supplies the medical images to the GPU 305ᵢ through the PCI switch 302 if necessary.

The GPU 305ᵢ performs image processing of a medical image supplied through the PCI switch 302 and outputs a resulting medical image to the CPU 303 through the PCI switch 302.

The CPU 303 stores the medical image outputted from the GPU 305ᵢ into the memory 304.

Supply of a medical image stored in the memory 304 to the GPU 305ᵢ, image processing of a medical image by the GPU 305ᵢ and storage of a medical image after image processing outputted from the GPU 305ᵢ into the memory 304 are repeated as necessary.

Then, after all necessary image processing is performed by the GPU 305ᵢ and a medical image after the image processing is stored into the memory 304, the CPU 303 reads out a medical image stored in the memory 304 and supplies the medical image to the camera input/output I/F 301 through the PCI switch 302.

The camera input/output I/F 301 supplies the medical image supplied from the CPU 303 after all necessary image processing has been performed to the display apparatus 202.

### <Example of image processing performed by CCU 201>

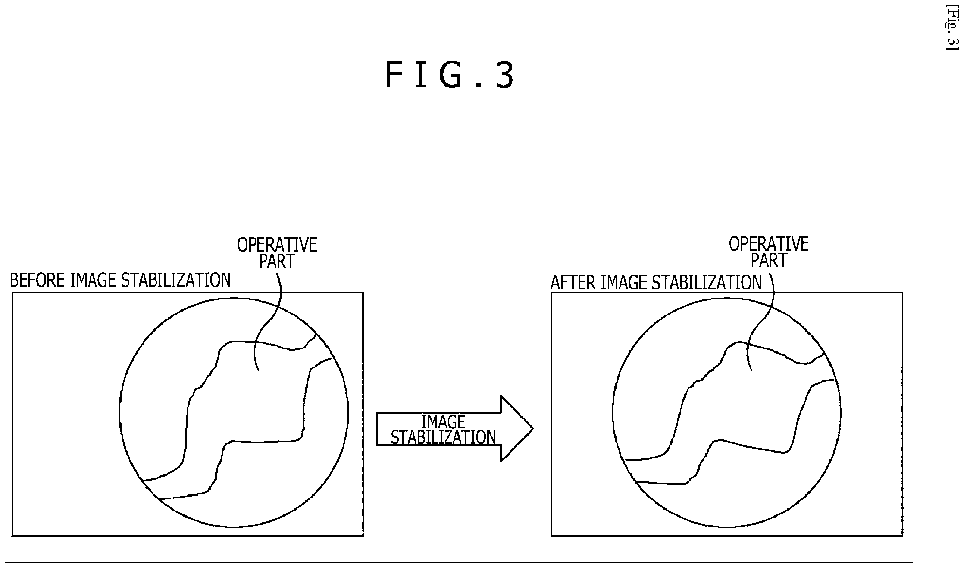

FIG. 3 is a view illustrating an outline of image stabilization (process) as an example of image processing performed for a medical image by the CCU 201.

In the image stabilization, an image pickup object (image) of an operative part or the like appeared on a medical image is deformed so as to cancel the image shake. In FIG. 3, as deformation of a medical image as the image stabilization, an image pickup object appeared on a medical image is rotated by a predetermined angle in the clockwise direction and besides is translated parallelly by a predetermined distance in the leftward direction.

If a user such as a doctor or a scopist performs image pickup while holding the endoscope 100 in hand, a medical image picked up by the endoscope 100 becomes a blurred image arising from the fact that the endoscope 100 is shaken, and such a blurred medical image as just described may possibly be displayed on the display apparatus 202. By the image stabilization, blurring of an image pickup object appeared on a medical image can be suppressed and a medical image that can be easily seen by a user can be displayed.

It is to be noted that deformation of a medical image as image stabilization can be performed, for example, by affine transformation.

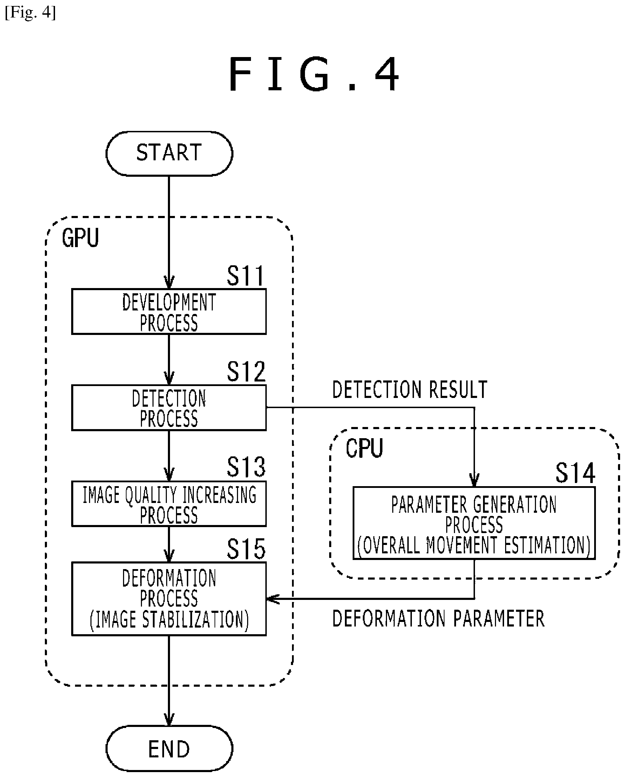

FIG. 4 is a flow chart illustrating an outline of an example of image processing including image stabilization performed for a medical image by the CCU 201.

At step S11, the GPU 305ᵢ performs a development process for a medical image picked up by the endoscope 100, and the processing advances to step S12.

At step S12, the GPU 305ᵢ performs a detection process for the medical image after the development process, and the processing advances to steps S13 and S14. Here, in the detection process of the medical image, a movement amount for each one or more pixels of the medical image and other feature amounts are detected. The movement amount and so forth as a result of the detection process (detection result) are supplied from the GPU 305ᵢ to the CPU 303 through the PCI switch 302.

At step S13, the GPU 305ᵢ performs an image quality increasing process such as noise reduction for the medical image after the detection process.

At step S14, the CPU 303 estimates a movement of the overall medical image (overall screen image) in response to the detection result from the GPU 305ᵢ and performs a parameter generation process for generating deformation parameters to be used for deformation of the medical image as the image stabilization in response to the estimated movement. In the parameter generation process, for example, elements of a matrix for performing affine transformation as the image stabilization are generated as the deformation parameters. The CPU 303 supplies the deformation parameters to the GPU 305ᵢ through the PCI switch 302.

It is to be noted that, while the parameter generation process at step S14 here is performed by the CPU 303, the parameter generation process can be performed not by the CPU 303 but by the GPU 305ᵢ.

After steps S13 and S14, the processing advances to step S15, at which the GPU 305ᵢ performs image stabilization by performing a deformation process for deforming the medical image after the image quality increasing process in accordance with the deformation parameters supplied from the CPU 303.

The medical image after the image stabilization is supplied from the camera input/ output I/F 301 to and displayed on the display apparatus 202.

It is to be noted that the detection process at step S12 can be performed at an arbitrary timing before the deformation process at step S14, such as immediately before the development process at step S1 or immediately after the image quality increasing process at step S13. Further, the image quality increasing process at step S13 can be performed after the deformation process at step S15.

Incidentally, since, in FIG. 2, the CCU 201 includes a plurality of, that is, three, GPUs 305₁ to 305₃, image processing for a medical image can be performed by parallel (distributed) processing using the three GPUs 305₁ to 305₃. By performing the image processing for a medical image by parallel processing, processes from image pickup to display of a medical image can be performed in low latency.

For example, to put it simply, if the medical image is divided equally into a number of regions equal to the number of the GPUs 305₁ to 305₃, that is, equally into three regions, which are lined up in the horizontal direction and one GPU 305ᵢ is responsible for image processing (of an image) of each one region, then the time period required for the image processing can be reduced roughly to one third in comparison with that in an alternative case in which a single GPU is responsible for the image processing of the full medical image that is not divided.

However, in such a case that image processing in which a necessary region (range) of a medical image is unknown upon starting of image processing like deformation (processing) as image stabilization is to be performed for the medical image, the efficiency in parallel processing sometimes degrades significantly.

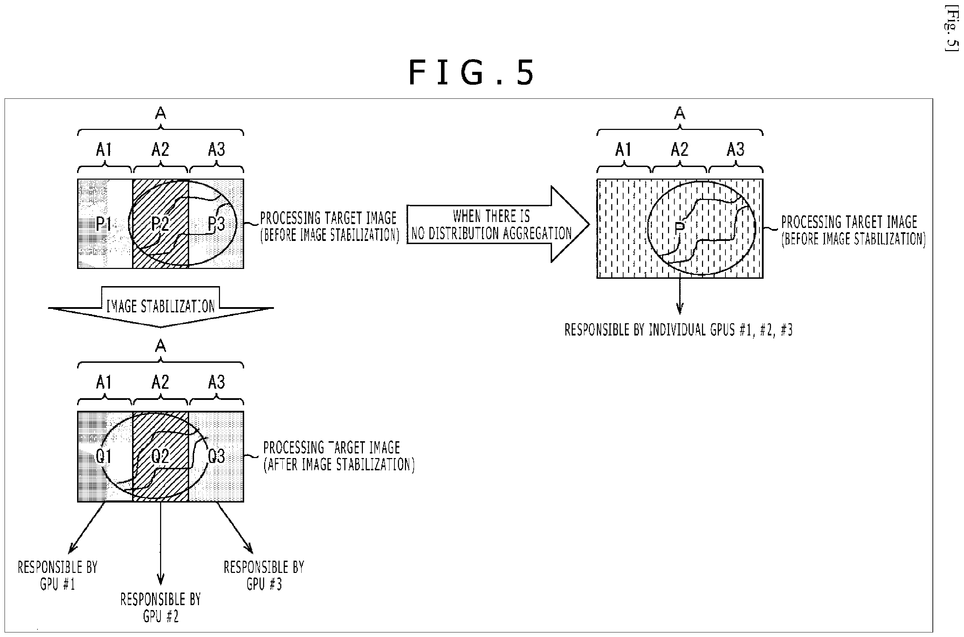

FIG. 5 is a view illustrating that, upon starting of image processing, the efficiency of parallel processing is degraded significantly when image processing in which a necessary region of a medical image is unknown.

Here, in the following description, as image processing to be performed for a medical image, for example, image processing including image stabilization is adopted. Further, the image processing is performed by setting, for example, one picture (frame) of a medical image as a processing target image of a processing target. Further, in the following description, the GPU 305₁, 305₂ and 305₃ are referred to also as GPU #1, GPU #2 and GPU #3, respectively.

It is to be noted that the image processing to be performed for a medical image is not limited to image processing that includes image stabilization.

Now, it is assumed that, for example, as depicted in FIG. 5, a medical image of one picture as a processing target image is equally divided into a number of regions equal to the number of GPU #1 to GPU #3 that are to perform parallel processing, that is, into three regions A1, A2 and A3, lined up in the horizontal direction and, setting the region A#i of the processing target image after the image stabilization as a responsible region A#i of the GPU #i, the GPU #i is responsible for outputting of an image Q#i of the responsible region A#i (here i = 1, 2, 3).

Here, if the GPU #i performs deformation in which the deformation amount is not zero as the image stabilization, then the image P#i of the responsible region A#i of the processing target image before the image stabilization and the image Q#i of the responsible region A#i of the processing target image after the image stabilization do not coincide with each other.

The GPU #i generates an image Q#i of the responsible region A#i of the processing target image after the image stabilization by deforming the processing target image before the image stabilization in accordance with the deformation parameters in order to output the image Q#i of the responsible region A#i.

Upon generation of the image Q#i of the responsible region A#i, the target region that becomes a target of deformation in accordance with the deformation parameters in the processing target image before the image stabilization is a region that becomes the responsible region A#i when the target region is deformed in accordance with the deformation parameters and is unknown before the deformation parameters are determined (generated).

Since, in FIG. 4 described hereinabove, generation of deformation parameters is performed in parallel to the image quality increasing process after the development process and the detection process, at the worst case, deformation parameters are determined after the development process, detection process and image quality increasing process.

Accordingly, deformation parameters and a target region that becomes a target of deformation in accordance with the deformation parameters are unknown before the development process, detection process and image quality increasing process come to an end.

On the other hand, although the GPU #i has a built-in memory and suitably stores an image into the built-in memory to perform image processing, the memories built in the GPU #1 to the GPU #3 are independent of each other, and (direct) exchange of data between the GPU #1 , the GPU #2 and the GPU #3 is not performed.

Accordingly, it is necessary for each GPU #i to have an image of a target region, which becomes a target of deformation in accordance with deformation parameters, stored in the built-in memory from within the processing target image before the image stabilization.

Since the target region is unknown before the development process, detection process and image quality increasing process come to an end, it is necessary for each GPU #i to have an overall processing target image before image stabilization stored in the built-in memory such that, whichever region of the processing target image before the image stabilization becomes a target region, it can cope with this.

Accordingly, as depicted in FIG. 5, it is necessary for each of the GPU #1 to the GPU #3 to be responsible at least for the development process and the image quality increasing process of an image P of a full area A of the processing target image separately and independently of each other (the detection process of the image P of the full area A of the processing target image may be performed by one of the GPU #1 to the GPU #3).

As a result, each of the GPUs #1 to #3i comes to perform a development process and an image quality increasing process for the same processing target image, and the efficiency in parallel processing is degraded significantly.

Also in a case in which a maximum amount of deformation in accordance with deformation parameters is known because it is limited or the like, it is necessary to store an image in a region necessary for deformation from 0 to the known maximum amount from within a processing target image before image stabilization into the built-in memory and perform a development process and an image quality increasing process. Accordingly, the efficiency in parallel processing is significantly degraded similarly.

As a method for suppressing that the efficiency in parallel processing is degraded significantly when image processing in which a necessary region of a processing target image is unknown upon starting of image processing like deformation as image stabilization as described above, a method is available, in which distribution aggregation is performed in which a necessary portion of a processing target image is distributed to the GPUs #i that perform parallel processing and (images of responsible regions A#i of) the processing target region after the image process by the GPUs #i is aggregated.

### <Distribution aggregation>

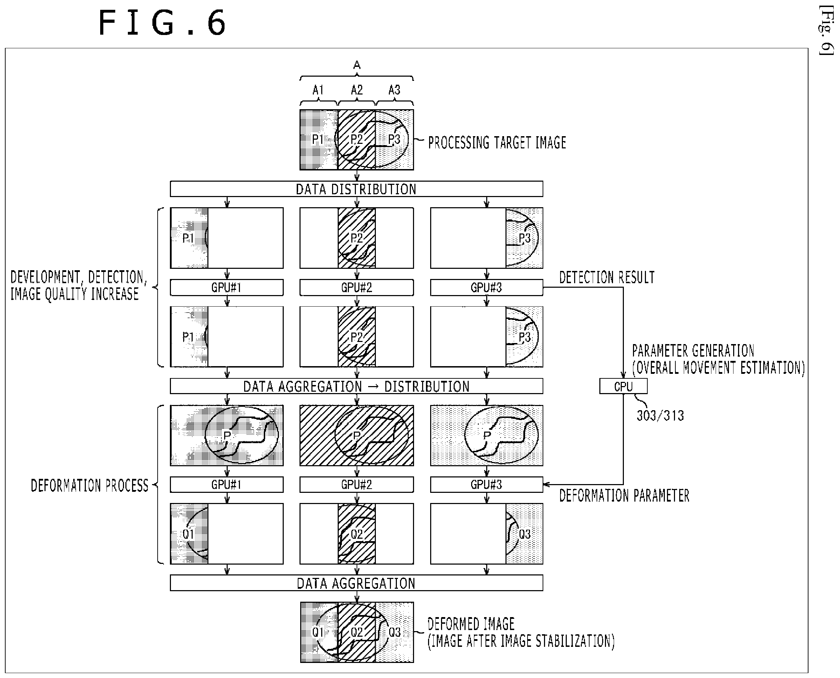

FIG. 6 is a view illustrating an example of distribution aggregation when image processing including the image stabilization described hereinabove with reference to FIG. 4 is performed for a processing target image.

Here, in order to simplify the description in the following, it is assumed that, for example, as depicted in FIG. 5, a processing target image is equally divided into a number of regions equal to the number of GPU #1 to GPU #3 that are to perform parallel processing, that is, into three regions A1, A2 and A3, lined up in the horizontal direction and, setting the region A#i of the processing target image after the image stabilization as a responsible region A#i, the GPU #i is responsible for outputting of an image Q#i of the responsible region A#i.

It is to be noted that the responsible regions A1, A2 and A3 are not limited to regions of an equal size when a processing target image is equality divided. In other words, as the responsible regions A1, A2 and A3, for example, regions of different sizes can be adopted. Further, as the responsible regions A1, A2 and A3, for example, regions that partly overlap with each other can be adopted.

An image P#i of a region A#i of a processing target image is distributed to a GPU #i that is responsible for the region A#i. The GPU #i whose responsible region A#i is the region A#i to which the image is distributed performs a development process, a detection process and an image quality increasing process as the first image processing for the image P#i of the responsible region A#i.

Further, each GPU #i supplies a detection result of the detection process for the image P#i of the response region A#i to the CPU 303, and the CPU 303 generates deformation parameters using the detection results from the GPUs #i and supplies the deformation parameters to the GPUs #i.

The images of the responsible regions A#i after the first image processing of the GPUs #i are aggregated as an image P of the full area A of the processing target image, and the image P of the full area A after the aggregation is distributed to the GPUs #i.

Each GPU #i specifies, on the basis of the deformation parameters from the CPU 303, an image of a region necessary to generate an image Q#i after the image stabilization of the responsible region A#i from within the image P of the full area A of the processing target image aggregated after the first image processing as an image of a target region for deformation. Further, each GPU #i performs a deformation process for deforming an image of a target region in accordance with deformation parameters supplied from the CPU 303 as second image processing to determine an image Q#i after the image stabilization only for the responsible region A#i.

The images Q#i of the responsible regions A#i of the GPUs #i after the second image processing are aggregated as an image of the full area A of the processing target image, and the image of the full area A after the aggregation is outputted as a deformation image after the image stabilization from the CCU 201 to the display apparatus 202.

As described above, when distribution aggregation is performed, each GPU #i may perform a development process, a detection process and an image quality increasing process as the first image processing with an image P#i of a responsible region A#i set as a target and may perform a deformation process as the second image process with an image of a region necessary to generate an image Q#i after the image stabilization of the responsible A#i set as an image of a target region of deformation. Therefore, such significant degradation of the efficiency in parallel processing of the GPUs #1 to #3 as described hereinabove with reference to FIG. 5 can be suppressed.

It is to be noted that, in the example of the CCU 201 of FIG. 2, distribution aggregation described hereinabove with reference to FIG. 6 is performed by the CPU 303.

### <Processing by CCU 201 when distribution aggregation is performed by CPU 303>

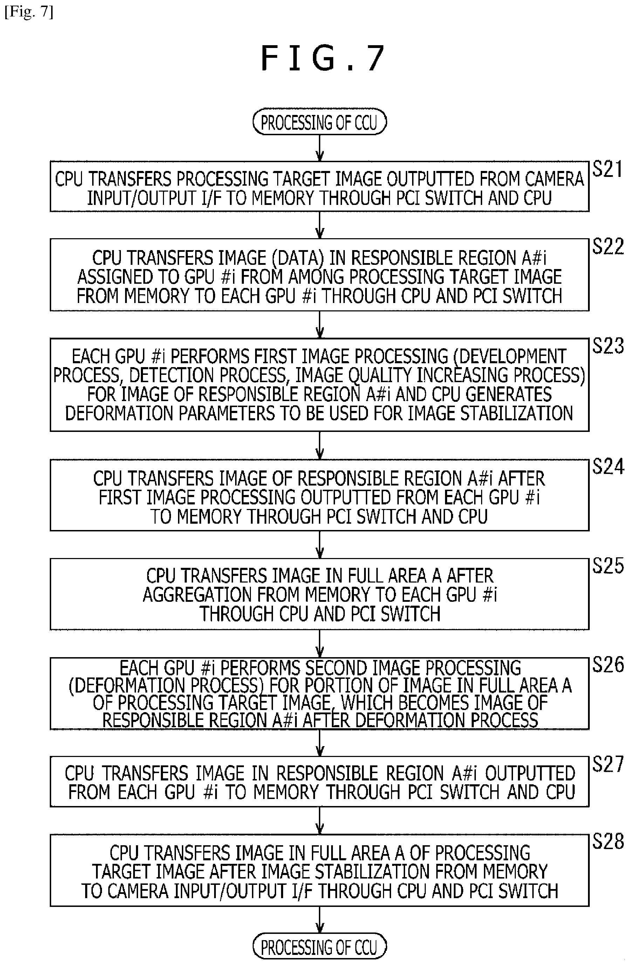

FIG. 7 is a flow chart illustrating an example of processing of the example of the CCU 201 of FIG. 2 when distribution aggregation is performed by the CPU 303.

It is to be noted that, in FIG. 7, it is assumed that a development process, a detection process and an image quality increasing process as the first image processing are performed and a deformation process as the second image processing is performed as described hereinabove with reference to FIG. 2.

A medical image picked up by the endoscope 100 is supplied to the camera input/ output I/F 301, and the camera input/output I/F 301 outputs the medical image from the endoscope 100 to the PCI switch 302.

At step S21, the CPU 303 transfers the medical image outputted from the camera input/output I/F 301 to the memory 304 through the PCI switch 302 and the CPU 303 so as to be stored into the memory 304 and sets one picture of the medical image stored in the memory 304 as a processing target image.

At step S22, the CPU 303 transfers an image (P#i) of the responsible region A#i for which each GPU #i is responsible from within the processing target image stored in the memory 304 from the memory 304 to the GPUs #i through the CPU 303 and the PCI switch 302 to distribute (the images of) the responsible regions A#i to the GPUs #i.

At step S23, each GPU #i performs, setting the image of the responsible region A#i as a target, a development process, a detection process and an image quality increasing process as the first image processing and outputs the image of the responsible region A#i after the first image processing to the PCI switch 302. Further, each GPU #i supplies a detection result of the detection process to the CPU 303 through the PCI switch 302. The CPU 303 generates deformation parameters to be used for deformation as the image stabilization in response to a detection result of each GPU #i and supplies the deformation parameters to the GPUs #is through the PCI switch 302.

At step S24, the CPU 303 transfers the images of the responsible regions A#i after the first image processing, the images outputted from the GPUs #i, to the memory 304 through the PCI switch 302 and the CPU 303 so as to be stored into the memory 304 thereby to aggregate the images of the responsible regions A#i after the first image processing of the GPUs #i as an image of the full area A of the processing target image.

At step S25, the CPU 303 transfers the image of the full area A of the processing target image after the aggregation, the image stored in the memory 304, to the GPUs #i through the CPU 303 and the PCI switch 302 to distribute the image of the full area A to the GPUs #i.

At step S26, each GPU #i sets a portion, which becomes an image of a responsible region A#i after the deformation process, of the image (P) of the full area A of the processing target image distributed from the CPU 303 as an image of a target region for deformation. Further, each GPU #i performs a deformation process for deforming the image of the target region in accordance with the deformation parameters from the CPU 303 as the second image processing to determine the image Q#i after the image stabilization and outputs the image Q#i to the PCI switch 302 only for the responsible region A#i.

At step S27, the CPU 303 transfers the images (Q#i) after the image stabilization of the responsible regions A#i, the images outputted from the GPUs #i, to the memory 304 through the PCI switch 302 and the CPU 303 so as to be stored into the memory 304 thereby to aggregate the images after the image stabilization of the responsible regions A#i of the GPUs #i as an image of the full area A of the processing target image after the image stabilization.

At step S28, the CPU 303 transfers the image of the full area A of the processing target image after the image stabilization, the image stored in the memory 304, from the memory 304 to the camera input/output I/F 301 through the CPU 303 and the PCI switch 302.

The processing target image after the image stabilization transferred to the camera input/output I/F 301 is supplied from the camera input/output I/F 301 to the PCI switch 302.

Incidentally, in the example of the CCU 201 of FIG. 2, when distribution aggregation is to be performed, the CPU 303 transfers an image that becomes a target of distribution or aggregation to the memory 304 managed by the CPU 303 so as to be stored into the memory 304.

Accordingly, even if the speed of processing of the GPUs #i is increased, the time period required for transfer of an image that becomes a target of distribution or aggregation to the memory 304 (transfer to the memory 304 and transfer from the memory 304) at least appears as latency arising from processes from image pickup to display of a medical image.

Therefore, the CCU 201 that can perform processes from image pickup to display of a medical image in lower latency is described below.

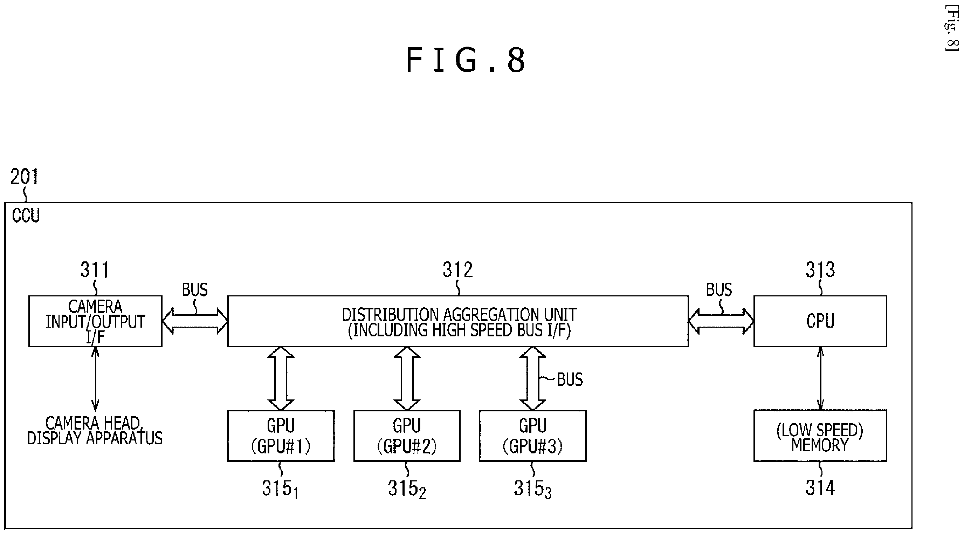

FIG. 8 is a block diagram depicting a configuration example of the CCU 201 of FIG. 1, which is an embodiment of the present invention.

Referring to FIG. 8, the CCU 201 is configured based on a PC architecture similarly to the case of FIG. 2 and includes a camera input/output I/F 311, a distribution aggregation unit 312 (switching control circuitry), a CPU 313 and a memory 314 as well as a plurality of, for example, three, GPUs 315₁, 315₂ and 315₃ (graphic processing circuits).

The camera input/output I/F 311 is an I/F for exchanging a medical image to and from the camera head 102 or the display apparatus 202 of FIG. 1, and supplies (image data of) a medical image picked up by the endoscope 100 and supplied from the camera head 102 to the distribution aggregation unit 312 and further supplies a medical image supplied from the distribution aggregation unit 312 to the display apparatus 202.

The distribution aggregation unit 312 is connected to the camera input/output I/F 311, CPU 313 and GPUs 315₁ to 3153 through a bus. The distribution aggregation unit 312 is one of high speed bus interfaces that can perform data transfer at a higher speed than a predetermined transfer speed such as, for example, a transfer speed of PCI or the like. For example, the distribution aggregation unit 312 relays exchange of a medical image or other data between the camera input/output I/F 311, CPU 313 and GPUs 315ᵢ, for example, in accordance with the PCIe (PCI express) standard.

Accordingly, the camera input/output I/F 311, distribution aggregation unit 312, CPU 313 and GPUs 315₁ to 315₃ have a built-in I/F of PCIe as a bus I/F for connection to the bus. The camera input/output I/F 311, CPU 313 and GPUs 315ᵢ are different from the camera input/output I/F 301, CPU 303 and GPUs 305ᵢ, respectively, of FIG. 2 that have an I/F of PCI as a bus I/F in that a high speed I/F of PCIe is built therein as a bus I/F.

Since, in FIG. 8, a bus I/F of PCIe higher in speed than PCI is adopted as described above, latency reduced by increase of the speed can be implemented.

Further, the distribution aggregation unit 312 performs distribution aggregation of images without involvement of the memory 314 (memory circuitry) managed by the CPU 313. In addition, the distribution aggregation unit 312 performs distribution aggregation of images independent of the CPU 313. In particular, the distribution aggregation unit 312 aggregates medical images outputted from the GPUs 315₁ to 315₃ and distributes a medical image to the respective GPUs 315₁ to 315₃ without involvement of the memory 314.

Furthermore, the distribution aggregation unit 312 distributes a medical image outputted from the camera input/output I/F 311 to the respective GPUs 315₁ to 315₃ and supplies a medical image after aggregation to the camera input/output I/F 311 without involvement of the memory 314.

As described above, in FIG. 8, the CPU 313 (303) does not perform distribution aggregation as in the case of FIG. 2, but the distribution aggregation unit 312 different from the CPU 313 preforms distribution aggregation.

The CPU 313 controls the entire CCU 201 in accordance with a predetermined program. For example, the CPU 313 controls distribution aggregation of the distribution aggregation unit 312. A program to be executed by the CPU 313 can be installed in advance into the memory 314, can be installed from a recording medium not depicted into the memory 314, can be downloaded from a site and installed into the memory 314 or the like.

The memory 314 stores necessary data under the management of the CPU 313. It is to be noted that, in FIG. 8, reading and writing of a medical image from and into the memory 314 is not performed. Accordingly, it is not necessary to adopt a high speed memory for the memory 314 from a point of view that processes from image pickup to display of a medical image are performed in low latency like the memory 304 of FIG. 2. In other words, as the memory 314, it is possible to adopt a memory that is, for example, lower in speed than the memory 304 of FIG. 2.

The GPU 315ᵢ (i = 1, 2, 3 in FIG. 8) is an example of an image processing unit that performs image processing for a medical image supplied from the distribution aggregation unit 312 and supplies a medical image after image processing to the distribution aggregation unit 312.

Although, here in FIG. 8, the three GPUs 315₁ to 315₃ are provided in the CCU 201, the number of GPUs is not limited to three. In particular, a plural number of 2 or 4 or more GPUs can be provided in the CCU 201.

In the CCU 201 configured in such a manner as described above, the camera input/ output I/F 311 outputs a medical image supplied from the camera head 102 to the distribution aggregation unit 312. The distribution aggregation unit 312 distributes the medical image outputted from the camera input/output I/F 311 to the GPUs 315ᵢ.

The GPU 315ᵢ performs image processing for the medical image distributed through the distribution aggregation unit 312 and outputs a resulting medical image to the distribution aggregation unit 312.

The distribution aggregation unit 312 aggregates medical images outputted from the GPUs 315ᵢ and outputs the image after the aggregation to the camera input/output I/F 311 or distributes the image after the aggregation to the GPUs 315ᵢ.

When the distribution aggregation unit 312 distributes the image after the aggregation to the GPUs 315ᵢ, the distribution and the aggregation of medical images after the image processing outputted from the GPUs 315ᵢ are repeated if necessary.

Then, after all necessary image processing is performed by the GPUs 315ᵢ and medical images after the image processing outputted from the GPUs 315ᵢ to the distribution aggregation unit 312, the distribution aggregation unit 312 aggregates the medical images outputted from the GPUs 315ᵢ and outputs an image after the aggregation to the camera input/output I/F 311.

The camera input/output I/F 311 supplies the medical image after the aggregation outputted from the distribution aggregation unit 312 to the display apparatus 202.

### <Configuration example of distribution aggregation unit 312>

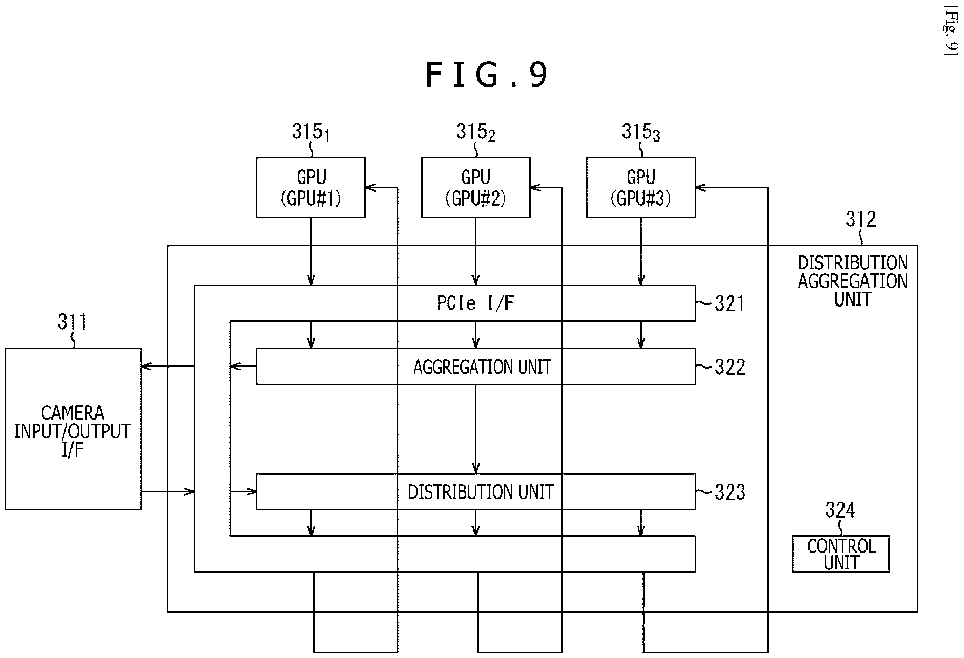

FIG. 9 is a block diagram depicting a configuration example of the distribution aggregation unit 312 of FIG. 8.

Referring to FIG. 9, the distribution aggregation unit 312 includes a PCIe I/F 321, an aggregation unit 322, a distribution unit 323 and a control unit 324 and is configured, for example, from an FPGA (Field-Programmable Gate Array).

The PCIe I/F 321 performs exchange of a medical image or other data to and from the camera input/output I/F 311 or the GPUs 315ᵢ. Then, the PCIe I/F 321 supplies a medical image outputted from the camera input/output I/F 311 to the distribution unit 323 and supplies a medical image outputted from the aggregation unit 322 to the camera input/output I/F 311. Further, the PCIe I/F 321 supplies a medical image outputted from the GPUs 315ᵢ to the aggregation unit 322 and supplies a medical image outputted from the distribution unit 323 to the GPUs 315ᵢ.

The aggregation unit 322 aggregates medical images supplied from the GPUs 315ᵢ through the PCIe I/F 321 and supplies a medical image after the aggregation to the distribution unit 323. Further, the aggregation unit 322 supplies the medical image after the aggregation to the camera input/output I/F 311 through the PCIe I/F 321.

The distribution unit 323 distributes a medical image supplied from the camera input/ output I/F 311 through the PCIe I/F 321 or a medical image after aggregation supplied from the aggregation unit 322 to the GPUs 315ᵢ through the PCIe I/F 321.

The control unit 324 controls a data flow of image processing of a medical image by controlling aggregation performed by the aggregation unit 322 and distribution performed by the distribution unit 323 under the control of the CPU 313.

### <Configuration example of GPU 315ᵢ>

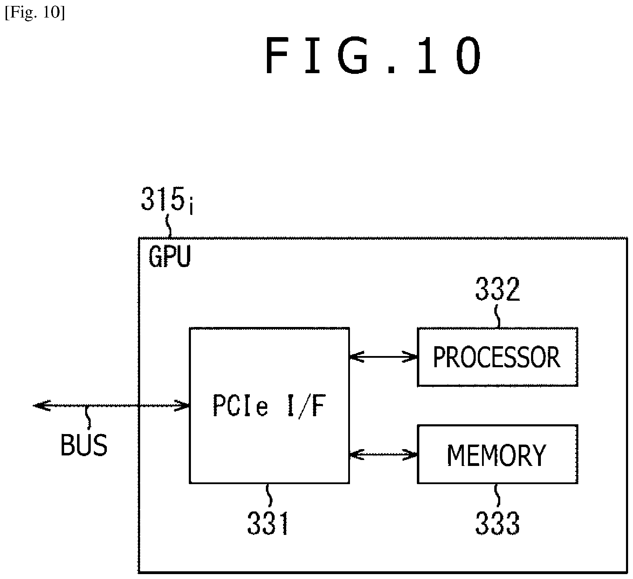

FIG. 10 is a block diagram depicting a configuration example of the GPU 315ᵢ of FIG. 8.

Referring to FIG. 10, the GPU 315ᵢ incudes a PCIe I/F 331, a processor 332 and a memory 333.

The PCIe I/F 331 performs exchange of a medical image or other data to and from the distribution aggregation unit 312 in accordance with the standard of PCIe. Then, the PCIe I/F 331 supplies a medical image outputted (distributed) from the distribution aggregation unit 312 to the processor 332 or the memory 333 and outputs a medical image after the process by the processor 332 is performed or a medical image stored in the memory 333 to the distribution aggregation unit 312.

The processor 332 performs predetermined image processing by executing a program for predetermined image processing. The memory 333 stores data necessary for operation of the processor 332.

### <Processing of CCU 201>

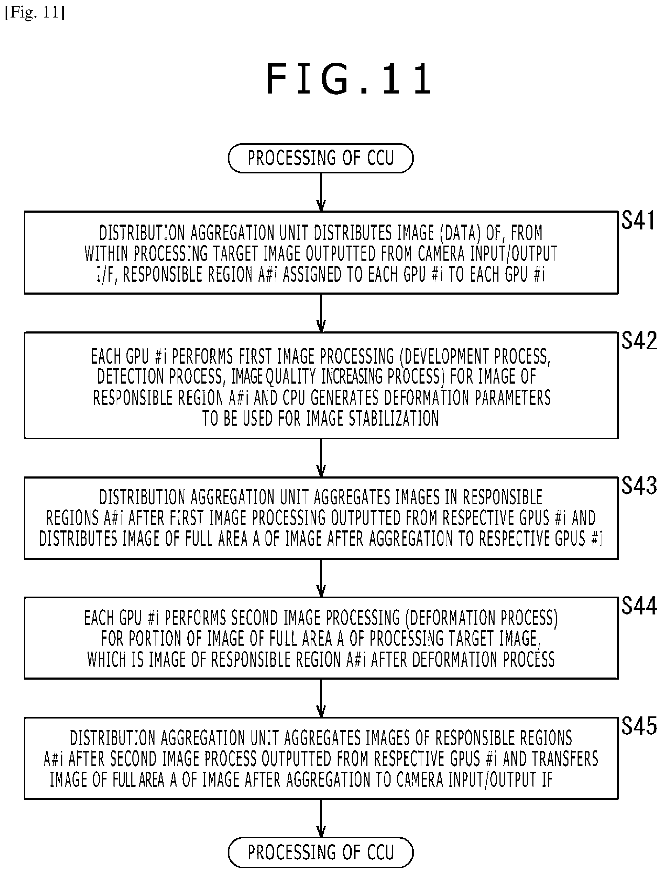

FIG. 11 is a flow chart illustrating an example of processing of the configuration example of the CCU 201 of FIG. 8.

In particular, FIG. 11 depicts an example of processing of the configuration example of the CCU 201 of FIG. 8 when image processing including image stabilization is performed for a medical image.

Here, as described hereinabove, the image processing to be performed for a medical image is not limited to the image processing that includes image stabilization.

Further, in the following description, the GPUs 315₁, 315₂ and 315₃ are referred to as GPU #1, GPU #2 and GPU #3, respectively.

Further, also in the following description, it is assumed that, as described hereinabove with reference to FIG. 5, a medical image of one picture as a processing target image is equally divided into a number of regions equal to the number of GPU #1 to GPU #3 that are to perform parallel processing, that is, into three regions A1, A2 and A3, lined up in the horizontal direction and, setting the region A#i of the processing target image after the image stabilization as a responsible region A#i of the GPU #i, the GPU #i is responsible for outputting of an image Q#i of the responsible region A#i.

It is to be noted that, as described hereinabove with reference to FIG. 6, the responsible regions A1, A2 and A3 are not limited to regions of an equal size when a processing target image is equality divided. In other words, as the responsible regions A1, A2 and A3, for example, regions of different sizes or regions that partly overlap with each other can be adopted.

A medical image picked up by the endoscope 100 is supplied to the camera input/ output I/F 311, and the camera input/output I/F 311 outputs the medical image from the endoscope 100 to the distribution aggregation unit 312.

At step S41, the distribution aggregation unit 41 sets one picture of a medical image outputted from the camera input/output I/F 311 as a processing target image. Further, the distribution aggregation unit 312 distributes images (P#i) of the responsible region A#i, for which the GPUs #i are responsible from within the processing target image to the GPUs #i.

At step S42, each GPU #i performs, setting an image of a responsible region A#i, a development process, a detection process and an image quality increasing process as the first image processing and outputs the image of the responsible region A#i after the first image processing to the distribution aggregation unit 312. Further, the GPU #i supplies a detection result of the detection process for the image of the responsible region A#i to the CPU 313 through the distribution aggregation unit 312. The CPU 313 generates deformation parameters to be used for deformation as image stabilization in response to the detection results from the GPUs #i and supplies the deformation parameters to the GPUs #i through the distribution aggregation unit 312.

At step S43, the distribution aggregation unit 312 aggregates the images of the responsible regions A#i after the first image processing, the images outputted from the GPUs #i, as an image of the full area A of the processing target image. Further, the distribution aggregation unit 312 distributes the image of the full area A of the processing target image after the aggregation to the GPUs #i.

At step S44, each of the GPUs #i sets a portion, which becomes an image of the responsible region A#i after the deformation process, of the image (P) of the full area A of the processing target image distributed from the distribution aggregation unit 312 as an image of a target region of deformation. Further, each of the GPUs #i performs a deformation process for deforming an image of the target region in accordance with the deformation parameters from the CPU 313 as the second image processing to determine an image Q#i after image stabilization only for the responsible region A#i and outputs the image Q#i to the distribution aggregation unit 312.

At step S45, the distribution aggregation unit 312 aggregates the images (Q#i) after the image stabilization of the responsible regions A#i, the images outputted from the GPUs #i, as an image of the full area A of the processing target image after the image stabilization. Then, the distribution aggregation unit 312 transfers the image after the aggregation, that is, the image of the full area A of the processing target image after the image stabilization, to the camera input/output I/F 311.

The processing target image after the image stabilization transferred from the distribution aggregation unit 312 to the camera input/output I/F 311 is supplied from the camera input/output I/F 311 to the display apparatus 202.

In this manner, in the configuration example of the CCU 201 of FIG. 8, the distribution aggregation unit 312 performs distribution aggregation without involvement of the memory 314 managed by the CPU 313. Therefore, the transfer time period for the memory 314 (304) is eliminated, and processes from pickup to display of a medical image can be performed in reduced latency.

### <Timing of processing>

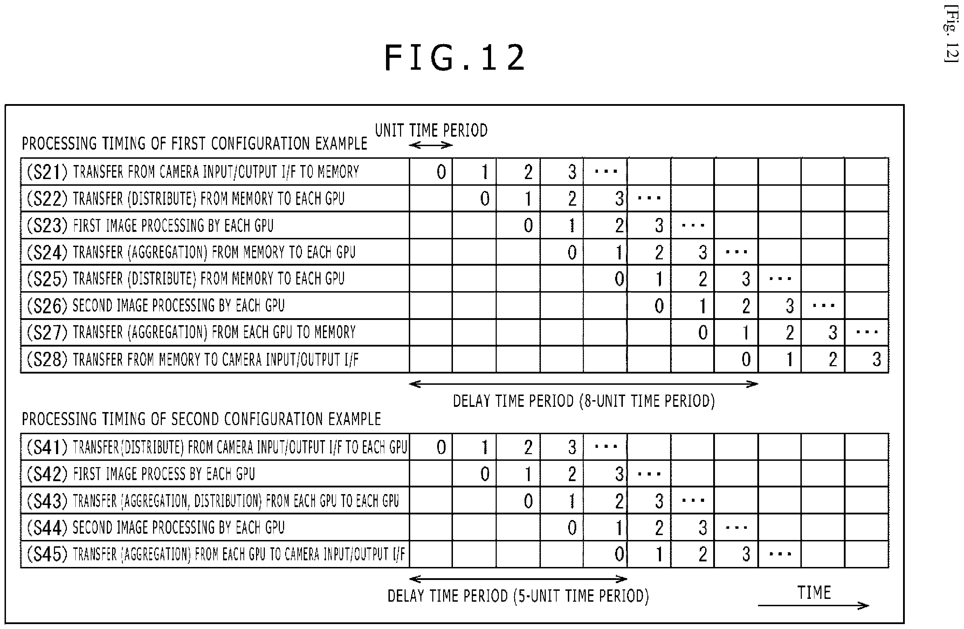

FIG. 12 is a view illustrating an example of timings of processing of the example of the CCU 201 of FIG. 2 and the configuration example of the CCU 201 of FIG. 8.

Here, it is assumed that, in the examples of the CCU 201, one step described hereinabove with reference to the flow charts of FIGS. 7 and 11 is performed in a predetermined unit time period.

Further, it is assumed that, to frames of a medical image, integral numbers are applied as frame numbers in an ascending order from an initial value set to 0.

FIG. 12 depicts a relationship between the respective steps in the flow charts of FIGS. 7 and 11 and frame numbers of a medical image that is a processing target image for which processing is to be performed at the steps.

As depicted in FIG. 12, in the example of the CCU 201, the period of time after the process at step S21 is started until the process at step S28 is ended for frames whose frame number is represented by f (f = 0, 1, 2, ...) is an 8-unit time period. In contrast, in the configuration example of the CCU 201, the period of time after the process at step S41 is started until the process at step S45 is ended for frames whose frame number is represented by f is a 5-unit time period that is reduced by an amount by which transfer to the memory 314 (304) is not involved.

Accordingly, with the configuration example of the CCU 201, latency reduced by a 3 (= 8 - 5) units time period from that in the example of the CCU 201 can be implemented.

### <Control of data flow>

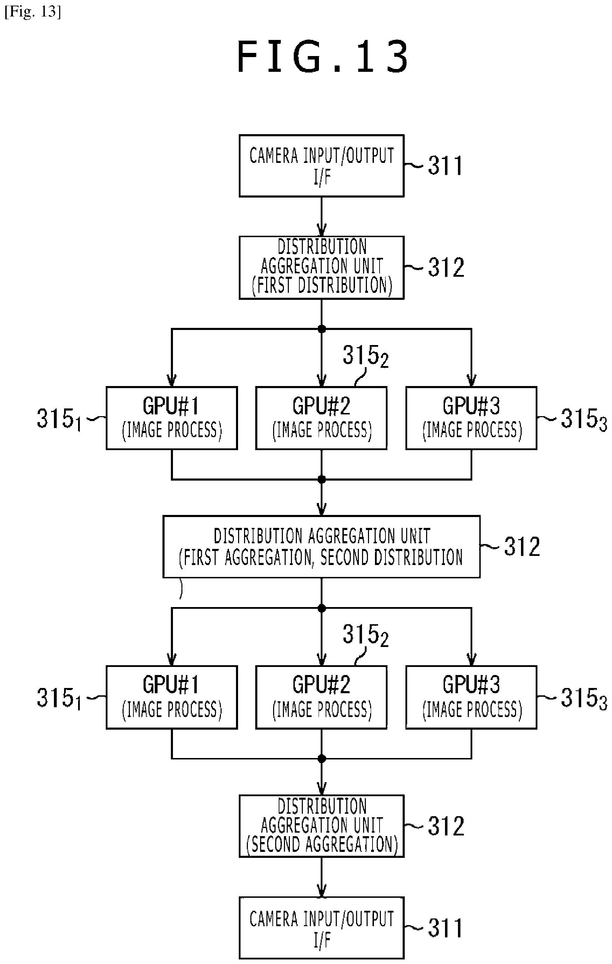

FIG. 13 is a view depicting a first example of a data flow when processing is performed by the configuration example of the CCU 201 of FIG. 8.

In particular, FIG. 13 depicts a data flow when processing in accordance with the flow chart of FIG. 11 is performed by the configuration example of the CCU 201.

In the first example of the data flow of FIG. 13, a processing target image outputted from the camera input/output I/F 311 is distributed to each of the three GPUs #1 to #3, by which image processing is performed for the processing target image distributed thereto in each of the GPUs #1 to #3. Then, the processing target image after the image processing is outputted from each of the GPUs #1 to #3 to the distribution aggregation unit 312.

The distribution aggregation unit 312 aggregates the processing target images after the image processing outputted from the GPUs #1 to #3, and the processing target image after the aggregation is distributed to each of the GPUs #1 to #3. The GPUs #1 to #3 individually perform image processing for the processing target image distributed thereto, and the processing target image after the image processing is outputted to the distribution aggregation unit 312.

The distribution aggregation unit 312 aggregates the processing target images after the image processing outputted from the respective GPUs #1 to #3, and the processing target image after the aggregation is outputted to the camera input/output I/F 311.

Although, in the first example of the data flow of FIG. 13, distribution and aggregation are individually performed twice, the number of times of distribution and aggregation is not limited to two but may be one or three or more.

Further, in the first example of the data flow of FIG. 13, in both of the first time distribution and the second time distribution, the processing target image is distributed to all of the three GPUs #1 to #3. However, as a GPU of the distribution destination, an arbitrary one or more ones of the three GPUs #1 to #3 may be adopted. Further, between the first time distribution and the second time distribution, the GPU or GPUs adopted as the distribution destination may be changed.

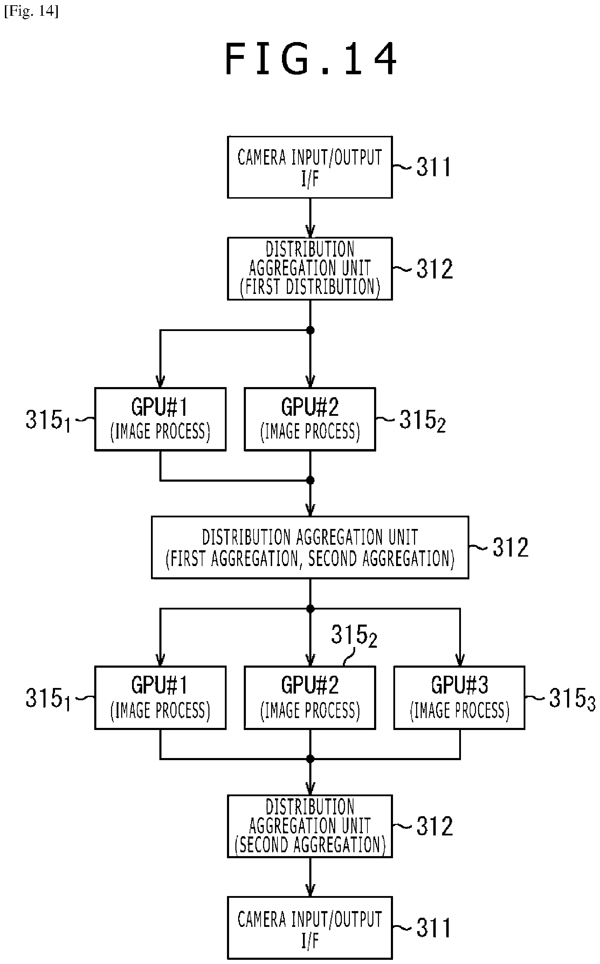

FIG. 14 is a view depicting a second example of the data flow when processing is performed by the configuration example of the CCU 201 of FIG. 8.

In the second example of the data flow of FIG. 14, distribution and aggregation are individually performed twice similarly as in the case of the first example of the data flow of FIG. 14.

However, in FIG. 14, in the first time distribution, the distribution destination is two GPUs #1 and #2 from among the three GPUs #1 to #3, and in the second time distribution, the distribution destination is the three GPUs #1 to #3. Accordingly, the first time aggregation is performed for outputs of the two GPUs #1 and #2, and the second time aggregation is performed for outputs of the three GPUs #1 to #3.

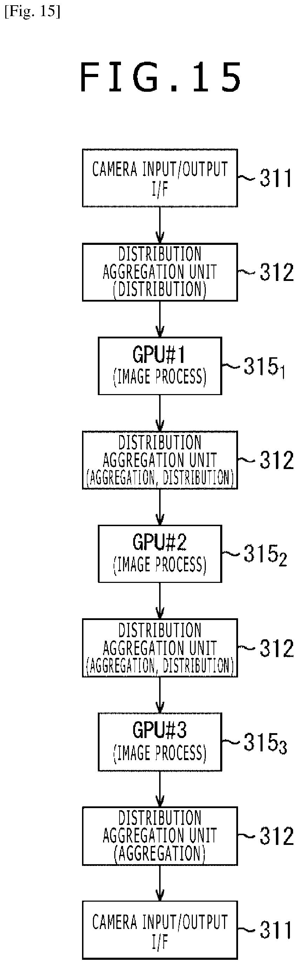

FIG. 15 is a view depicting a third example of the data flow when processing is performed by the configuration example of the CCU 201 of FIG. 8.

In the third example of the data flow of FIG. 15, distribution and aggregation are individually performed three times. One distribution destination in each of the first to third time distributions individually is one of the GPUs #1 to #3, and each of the first to third time aggregations is performed individually for one of the GPUs #1 to #3. Since, in FIG. 15, each aggregation is performed for an output of one GPU #i, the image after the aggregation is equal to an output of one GPU #i of the target of the aggregation, and in the aggregation, for example, the output of one GPU #i of the target of the aggregation becomes as it is as an image after the aggregation.

In the distribution aggregation unit 312 (FIG. 9), the control unit 324 controls the GPUs to be made a distribution destination of distribution by the distribution unit 323, (outputs of) the GPUs to be made a target of aggregation by the aggregation unit 322 and the number of times of aggregation by the aggregation unit 322 and distribution by the distribution unit 323, under the control of the CPU 313. Consequently, by dynamically changing the data flows in image processing of a medical image performed by the CCU 201, the data flows depicted in FIGS. 13 to 15 and other data flows can be implemented readily.

### <Processing target image>

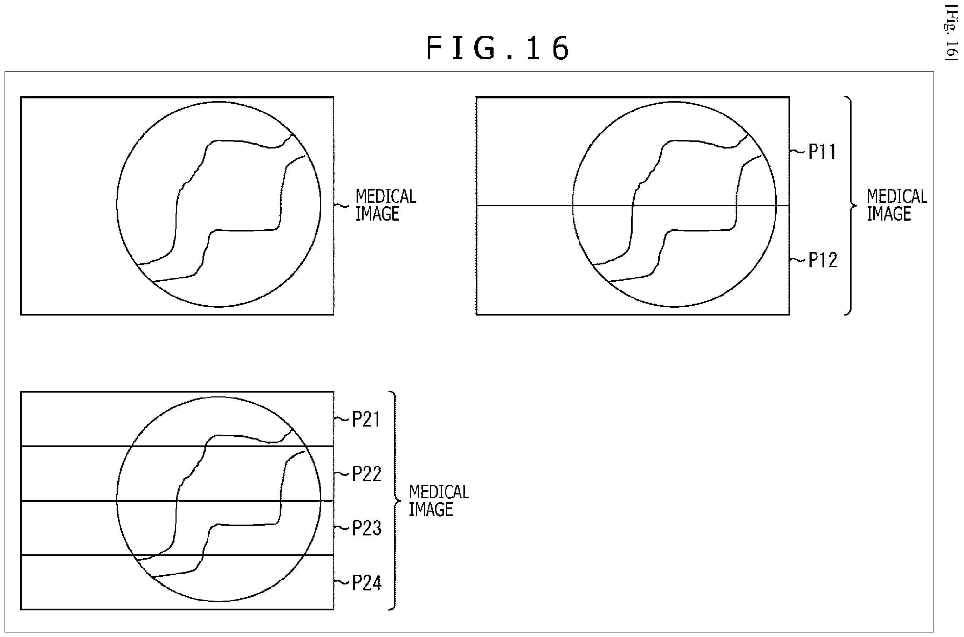

FIG. 16 is a view depicting an example of a processing target image that is made a processing target by the CCU 201.

The CCU 201 can determine an entire medical image, for example, of one picture as a processing target image as depicted in FIG. 16.

Further, the CCU 201 can determine each of images P11 and P12 obtained by dividing, for example, a medical image of one picture vertically into two as processing target images.

Furthermore, the CCU 201 can determine each of images P21, P22, P23 and P24 obtained by dividing, for example, a medical image of one picture vertically into four as processing target images.

Further, the CCU 201 can determine each of images obtained by dividing a medical image of one picture vertically into an arbitrary number as a processing target image.

Then, the CCU 201 can divide the processing target image into a number of regions lined up in the horizontal direction, the number being three equal to the number of the GPUs #1 to #3 in the maximum. Thus, in the CCU 201, the GPUs #1 to #3 can perform image processing for the respective regions by parallel processing.

### <Different configuration example of distribution aggregation unit 312>

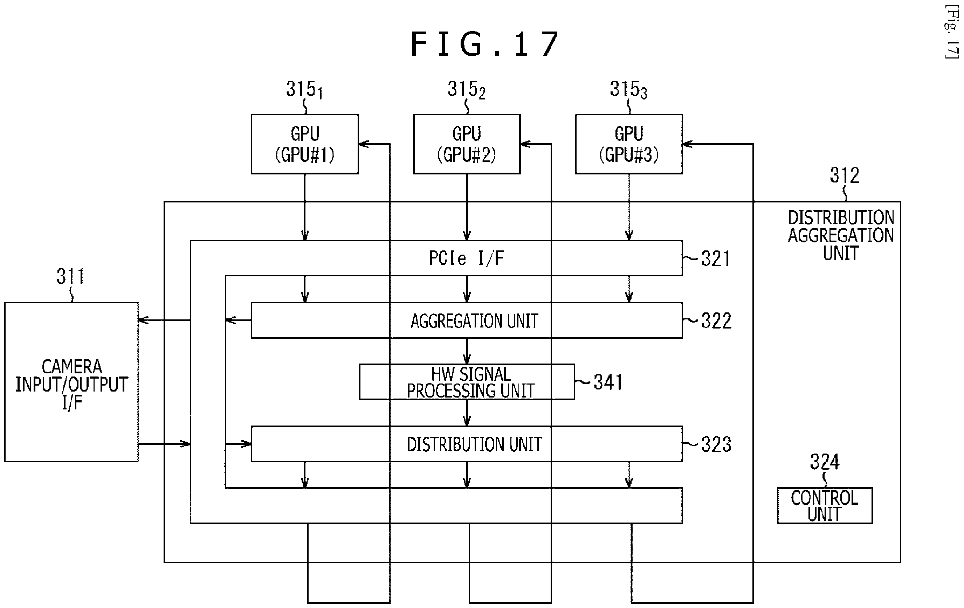

FIG. 17 is a block diagram depicting another configuration example of the distribution aggregation unit 312 of FIG. 8.

It is to be noted that, in FIG. 17, portions corresponding to those of FIG. 9 are denoted by like reference characters, and in the following description, description of them is omitted suitably.

The distribution aggregation unit 312 of FIG. 17 includes a PCIe I/F 321, an aggregation unit 322, a distribution unit 323, a control unit 324 and an HW (Hardware) signal processing unit 341 and is configured, for example, from an FPGA.

Accordingly, the distribution aggregation unit 312 of FIG. 17 is common to that in the case of FIG. 9 in that it includes the components from the PCIe I/F 321 to the control unit 324. However, the distribution aggregation unit 312 of FIG. 17 is different from that of FIG. 9 in that the HW signal processing unit 341 is provided newly.

The HW signal processing unit 341 performs predetermined signal processing for a processing target image (medical image) outputted from the aggregation unit 322 and outputs the processing target image after the predetermined signal processing to the HW signal processing unit 341.

Accordingly, in FIG. 17, the HW signal processing unit 341 performs distribution of a processing target image outputted from the HW signal processing unit 341.

As the predetermined signal processing to be performed by the HW signal processing unit 341, arbitrary signal processing can be adopted. For example, part of image processes performed by the GPU #i can be adopted as the predetermined signal processing to be performed by the HW signal processing unit 341.

Since the distribution aggregation unit 312 includes the HW signal processing unit 341 such that the HW signal processing unit 341 is responsible for part of signal processes to be performed by the CCU 201, it is possible to implement acceleration of image processing to be performed by the CCU 201 and implement further reduction in latency.

It is to be noted that the HW signal processing unit 341 can output a processing target image outputted from the aggregation unit 322 as it is without especially performing signal processing to the HW signal processing unit 341 if necessary. Whether the HW signal processing unit 341 is to perform the signal processing can be controlled by the control unit 324 (FIG. 17), and the data flow of the image processing to be performed by the CCU 201 can be controlled by the control of the control unit 324.

Further, the HW signal processing unit 341 can perform signal processing for a processing target image outputted from the distribution unit 323 and supply the processing target image after the signal processing to the GPUs #i.

### <Third configuration example of CCU 201>

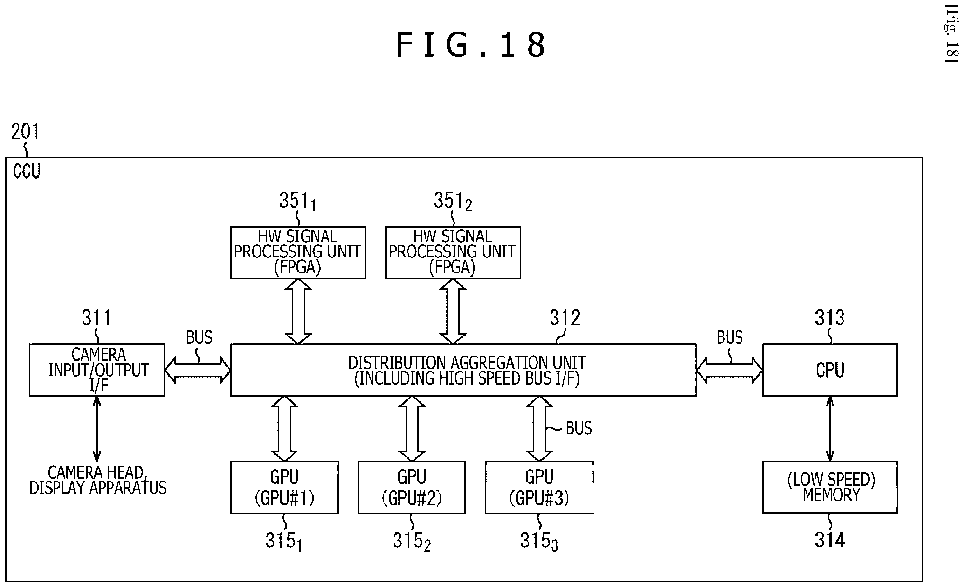

FIG. 18 is a block diagram depicting a third configuration example of the CCU 201 of FIG. 1.

It is to be noted that, in FIG. 18, portions corresponding to those of FIG. 8 are denoted by like reference characters, and in the following description, description of them is omitted suitably.

Referring to FIG. 18, the CCU 201 is configured based on a PC architecture similarly as in the case of FIG. 8 and includes a camera input/output I/F 311, a distribution aggregation unit 312, a CPU 313, a memory 314, GPUs 315₁, 315₂ and 315₃, and a plurality of, for example, two, HW signal processing units 351₁ and 351₂.

Accordingly, the CCU 201 of FIG. 18 is common to that of FIG. 8 in that it includes the components from the camera input/output I/F 311 to the GPU 315₃. However, the CCU 201 of FIG. 18 is different from that of FIG. 8 in that the HW signal processing units 351₁ and 351₂ are provided newly.

In FIG. 18, the distribution aggregation unit 312 supplies a processing target image after aggregation to HW signal processing units 351ⱼ if necessary.

The HW signal processing unit 351ⱼ (in FIG. 18, j = 1, 2) performs predetermined signal processing for a medical image as a processing target image supplied from the distribution aggregation unit 312 and supplies the processing target image after the predetermined signal processing to the distribution aggregation unit 312. Accordingly, after aggregation of the processing target image but before distribution of the processing target image after the aggregation, the HW signal processing unit 351ⱼ performs predetermined signal processing for the processing target image after the aggregation.

The HW signal processing unit 351ⱼ can be configured from hardware for exclusive use for performing, for example, FPGA and other specific signal processing and perform the predetermined signal processing at a high speed. In this case, it is possible to implement acceleration of image processing to be executed by the CCU 201 in reduced latency.

Here, while, in FIG. 18, the CCU 201 includes the two HW signal processing units 351₁ and 351₂, the number of HW signal processing units 351ⱼ is not limited to two. In particular, an arbitrary number of HW signal processing units 351ⱼ such as one, two or four or more HW signal processing units 351ⱼ may be provided in the CCU 201.

Further, as the predetermined signal processing to be performed by the HW signal processing units 351ⱼ, arbitrary signal processing can be adopted. For example, part of signal processing performed by the GPU #i can be adopted as the predetermined signal processing to be performed by the HW signal processing units 351ⱼ.

Further, the distribution aggregation unit 312 can output a processing target image after aggregation to the HW signal processing units 351ⱼ or output part of a processing target image by distribution of the processing target image, under the control of the control unit 324 (FIG. 9 or FIG. 17).

For example, if the distribution aggregation unit 312 (FIG. 17) outputs a processing target image after aggregation by the aggregation unit 322 to the HW signal processing unit 351ⱼ and determines the processing target image after signal processing outputted from the HW signal processing unit 351ⱼ as a target of distribution to the GPUs #i by the distribution unit 323, then the HW signal processing unit 351ⱼ performs, after aggregation of the processing target image but before distribution of the processing target image after the aggregation, the predetermined signal processing for the processing target image after the aggregation, similarly to the HW signal processing unit 341 of FIG. 17.

In the distribution aggregation unit 312, outputting (supply) of the processing target image to the HW signal processing unit 351ⱼ can be controlled by the control unit 324 (FIGS. 9 and 17), and the data flow of image processing to be performed by the CCU 201 can be controlled by the control of the control unit 324.

It is to be noted that the present technology can be applied not only to an endoscopic surgery system but also an electronic microscope and an arbitrary apparatus for processing a medical image. Further, the present technology can be applied not only to an apparatus that processes a medical image but also to an apparatus that processes an arbitrary image.

### <Description of computer to which present technology can be applied>

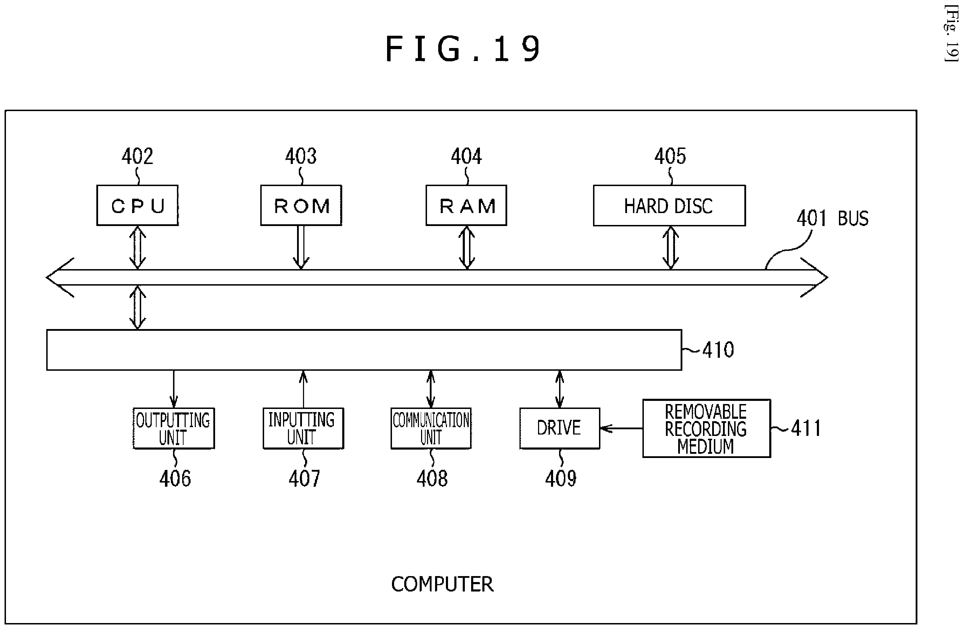

FIG. 19 is a block diagram depicting a configuration example of an embodiment of a computer to which the present technology can be applied.

The computer has a CPU 402 built therein, and an input/output interface 410 is connected to the CPU 402 through a bus 401.

If an inputting unit 407 is operated by a user to input an instruction to the CPU 402 through the input/output interface 410, then the CPU 402 executes a program stored in a ROM (Read Only Memory) 403 in accordance with the instruction. Alternatively, the CPU 402 loads a program stored in a hard disc 405 into a RAM (Random Access Memory) 404 and executes the program.

Consequently, the CPU 402 performs predetermined processing. Then, if necessary, the CPU 402 causes a result of the processing to be outputted from an outputting unit 406, to be transmitted from a communication unit 408, to be recorded on the hard disc or the like, for example, through the input/output interface 410.

It is to be noted that the inputting unit 407 is configured from a keyboard, a mouse, a microphone and so forth. Meanwhile, the outputting unit 406 is configured from an LCD (Liquid Crystal Display), a speaker and so forth.

Further, a program to be executed by the CPU 402 can be recorded in advance in the hard disc 405 or the ROM 403 as a recording medium built in the computer.

Further, the program can be stored (recorded) in a removable recording medium 411. Such a removable recording medium 411 as just described can be provided as package software. Here, as the removable recording medium 411, for example, a flexible disc, a CD-ROM (Compact Disc Read Only Memory), an MO (Magneto Optical) disc, a DVD (Digital Versatile Disc), a magnetic disc and a semiconductor memory are available.

Further, in addition to installation of the program into the computer from such a removable recording medium 411 as described hereinabove, the program can be downloaded into the computer through a communication network or a broadcasting network and installed into the hard disc 405 built in the computer. In particular, the program can be transferred, for example, from a download site to the computer by wireless communication through an artificial satellite for digital satellite broadcasting or can be transferred by wired communication to the computer through a network such as a LAN (Local Area Network) or the Internet.

In the computer of FIG. 19, a camera input/output I/F 311, a distribution aggregation unit 312 and GPUs 315ᵢ as well as necessary HW signal processing units 351ⱼ are provided so as to function as the CCU 201.

Here, processes the computer performs in accordance with the program may not necessarily be performed in a time series in accordance with the order described in the flow charts. In other words, the processes performed in accordance with the program by the computer include processes executed in parallel or separately (for example, parallel processing or processing by an object).

Further, the program may be processed by a single computer (processor) or may be processed in a distributed manner by a plurality of computers. Further, the program may be transferred to and executed by a remote computer.

Further, in the present specification, the term system is used to signify an aggregation of a plurality of constituent elements (devices, modules (parts) and so forth) and it does not matter whether or not all of the constituent elements are accommodated in the same housing. Accordingly, a plurality of apparatus accommodated in separate housings and connected to each other through a network configured system, and also one apparatus that includes a plurality of modules accommodated in a single housing configures a system.

The CPU may be defined as having N1 core(s) and N1^{∗}M1 thread(s), where M1=1∼3, "core" is processing circuit, and "thread" is a minimum unit of information.

The GPU may be defined as having N2 core(s) and N2^{∗}M2 thread(s), where M2=100∼ and N2>10^{∗}N1 (i.e., GPU has at least more than 10 times the core of CPU). In addition, the GPU may be a dedicated graphics processor efficiently implementing graphics operations, such as 2D, 3D graphics operations and/or digital video related functions. A GPU may include special programmable hardware that performs graphics operations, e.g. blitter functions, polygon/3D rendering, pixel shading, texture mapping, and vertex shading. A GPU may fetch data from a frame buffer and blend pixels together to render an image back into the frame buffer for display. GPUs may also control the frame buffer and permit the frame buffer to be used to refresh a display. A GPU may perform graphics processing tasks in place of CPUs coupled to the GPU to output raster graphics images to display devices through display controllers. While a CPU consists of a few cores optimized for sequential serial processing, a GPU has a parallel architecture consisting of hundreds or more of smaller efficient cores designed for simultaneous handling of multiple tasks thereby performing parallel operations on multiple sets of data.

The FPGA can be defined as a logic circuit, for example, a logic formed by a language dedicated to hardware design standardized by IEEE such as VHDL and Verilog HDL. The FPGA has circuit information, and a content of signal processing for the input signal in FPGA is determined by the circuit information.

It is to be noted that the embodiment of the present technology is not limited to the embodiment described above and can be altered in various manners without departing from the subject matter of the present technology as defined in the appended claims.

For example, the present technology can assume a configuration of a crowd computer in which one function is shared and cooperatively processed by a plurality of apparatus through a network.

Further, the respective steps described with reference to the flow charts described hereinabove not only can be executed by one apparatus but also can be shared and executed by a plurality of apparatus.

Furthermore, where a plurality of processes are included in one step, the plurality of processes included in the one step may be executed by a single apparatus or may be shared and executed by a plurality of apparatus.

Further, the effects described herein are exemplary to the end and are not restrictive, and other effects may be involved.

### Reference Signs List

10 Endoscope
100 Endoscope
101 Lens barrel
102 Camera head
110 Surgical tool
111 Insufflation tube
112 Energy treatment tool
120 Supporting arm apparatus
131 Operator
132 Patient
133 Patient bed
200 Cart
201 CCU
202 Display apparatus
203 Light source apparatus
204 Inputting apparatus
205 Treatment tool controlling apparatus
206 Insufflation apparatus
207 Recorder
208 Printer
301 Camera input/output I/F
302 PCI switch
303 CPU
304 Memory
305₁ to 305₃ GPU
311 Camera input/output I/F
312 Distribution aggregation unit
313 CPU
314 Memory
315₁ to 315₃ GPU
321 PCIe I/F
322 Aggregation unit
323 Distribution unit
324 Control unit
331 PCIe I/F
332 Processor
333 Memory
341, 351₁, 351₂ HW signal processing unit
401 Bus
402 CPU
403 ROM
404 RAM
405 Hard disc
406 Outputting unit
407 Inputting unit
408 Communication unit
409 Drive
410 Input/output interface
411 Removable recording medium

## Claims

1. A surgical endoscope system (10), comprising:
a surgical endoscope (100) configured to generate medical image data; and
an image processing apparatus, including:
switching control circuitry (312) receiving the medical image data generated by the surgical endoscope and configured to perform distribution and aggregation;
a plurality of graphic processing circuits (315) configured to perform image processing on the medical image data received via distribution from the switching control circuitry;
central processing circuitry (313) connected to the switching circuitry and to the plurality of graphic processing circuits via the switching control circuitry; and
memory circuitry (314) managed by the central processing circuitry,
wherein results from the image processing on the image data performed by the plurality of graphic processing circuits (315) are aggregated by the switching control circuitry (312, 322),
**characterised in that** the aggregation of the results is independent of the memory circuitry (314) managed by the central processing circuitry (313) before the results are output to the memory circuitry (314) via the central processing circuitry (313).

2. The surgical endoscope system according to claim 1, wherein the image processing on image data performed by the plurality of graphic processing circuits includes distinct image processing performed by at least two of the plurality of graphic processing circuits based on an instruction from the switching control circuitry.

3. The surgical endoscope system according to claim 2, wherein the plurality of graphic processing circuits perform a first image processing and a second image processing on the medical image data,
wherein, in the first image processing, the plurality of graphic processing circuits perform image processing on different areas of the medical image data respectively.

4. The surgical endoscope system according to claim 3, wherein the switching control circuitry distributes the medical image data to the plurality of graphic processing circuits before the first image processing, aggregates the results of the first image processing, and outputs the aggregation data to the memory circuitry.

5. The surgical endoscope system according to claim 4, wherein the first image processing includes at least one from a group consisting of: a development process, a detection process and an image quality increasing process.

6. The surgical endoscope system according to claim 5, wherein central processing circuitry generates deformation parameters based on results of the first image processing.

7. The surgical endoscope system according to claim 6, wherein, in the second image processing, the plurality of graphic processing circuits perform image processing based on the deformation parameters.

8. The surgical endoscope system according to claim 7, wherein the switching control circuitry distributes a full area image as the aggregation data to the plurality of graphic processing circuits,
the plurality of graphic processing circuit perform the second image processing on a target region of the full image based on the deformation parameters, and
wherein the target region is determined by the deformation parameters.

9. The surgical endoscope system according to claim 3, wherein the second image processing includes affine transformation for image stabilization.

10. The surgical endoscope system according to claim 1, wherein the results from the image processing on the image data performed by the plurality of graphic processing circuits that are aggregated by the switching control circuitry are thereafter redistributed to the plurality of graphic processing circuits.

11. The surgical endoscope system according to claim 10, wherein processing performed by the plurality of graphic processing circuits after the redistribution of the image data is performed using a parameter received from the central processing circuitry and generated based on information transmitted to the central processing circuitry from the plurality of graphic processing circuits before the aggregation.

12. An image processing apparatus, comprising:
switching control circuitry (312, 322, 323) configured to perform distribution and aggregation;
a plurality of graphic processing circuits (315) configured to perform image processing on image data received via distribution from the switching control circuitry;
central processing circuitry (313) connected to the switching circuitry and to the plurality of graphic processing circuits via the switching control circuitry; and
a memory circuitry (314) managed by the central processing circuitry,
wherein results from the image processing on the image data performed by the plurality of graphic processing circuits is aggregated by the switching control circuitry,
**characterised in that** the aggregation of the results is performed independent of the memory circuitry managed by the central processing circuitry before the results are output to the memory circuitry via the central processing circuitry.

13. The image processing apparatus according to claim 11, wherein the image processing on image data performed by the plurality of graphic processing circuits includes distinct image processing performed by at least two of the plurality of graphic processing circuits based on an instruction from the switching control circuitry.

14. An image processing method, comprising:
performing, using a plurality of graphic processing circuits (315), image processing on medical image data generated by a surgical endoscope or microscope and received via distribution from switching control circuitry (312); and
aggregating, by the switching control circuitry (312, 322), results from the image processing on the image data performed by the plurality of graphic processing circuits,
**characterised in that** the aggregation of the results is performed independent of a memory (314) managed by a central processing circuitry (313) before the results are output to the memory via the central processing circuitry, the central processing circuitry connected to the switching circuitry and to the plurality of graphic processing circuits via the switching control circuitry.

15. The image processing apparatus according to claim 14, wherein the image processing on image data performed by the plurality of graphic processing circuits includes distinct image processing performed by at least two of the plurality of graphic processing circuits based on an instruction from the switching control circuitry.

## Patentansprüche

1. Chirurgisches Endoskopsystem (10), das Folgendes umfasst:
ein chirurgisches Endoskop (100), das konfiguriert ist, medizinische Bilddaten zu erzeugen; und
eine Bildverarbeitungsvorrichtung, die Folgendes enthält:
eine Schaltsteuerungs-Schaltungsanordnung (312), die die durch das chirurgische Endoskop erzeugten medizinischen Bilddaten empfängt und konfiguriert ist, eine Verteilung und eine Vereinigung auszuführen;
mehrere Graphikverarbeitungsschaltungen (315), die konfiguriert sind, eine Bildverarbeitung an den über die Verteilung von der Schaltsteuerungs-Schaltungsanordnung empfangenen medizinischen Bilddaten auszuführen;
eine Zentraleinheit (313), die mit der Schaltsteuerungs-Schaltungsanordnung und über die Schaltsteuerungs-Schaltungsanordnung mit den mehreren Graphikverarbeitungsschaltungen verbunden ist; und
eine Speicherschaltungsanordnung (314), die durch die Zentraleinheit gemanagt wird,
wobei die Ergebnisse von der durch die mehreren Graphikverarbeitungsschaltungen (315) ausgeführten Bildverarbeitung an den Bilddaten durch die Schaltsteuerungs-Schaltungsanordnung (312, 322) vereinigt werden,
**dadurch gekennzeichnet, dass** die Vereinigung der Ergebnisse von der durch die Zentraleinheit (313) gemanagten Speicherschaltungsanordnung (314) unabhängig stattfindet, bevor die Ergebnisse über die Zentraleinheit (313) an die Speicherschaltungsanordnung (314) ausgegeben werden.

2. Chirurgisches Endoskopsystem nach Anspruch 1, wobei die durch die mehreren Graphikverarbeitungsschaltungen ausgeführte Bildverarbeitung an den Bilddaten eine unterschiedliche Bildverarbeitung enthält, die durch wenigstens zwei der mehreren Graphikverarbeitungsschaltungen basierend auf einer Anweisung von der Schaltsteuerungs-Schaltungsanordnung ausgeführt wird.

3. Chirurgisches Endoskopsystem nach Anspruch 2, wobei die mehreren Graphikverarbeitungsschaltungen eine erste Bildverarbeitung und eine zweite Bildverarbeitung an den medizinischen Bilddaten ausführen,
wobei bei der ersten Bildverarbeitung die mehreren Graphikverarbeitungsschaltungen eine Bildverarbeitung an jeweils verschiedenen Bereichen der medizinischen Bilddaten ausführen.

4. Chirurgisches Endoskopsystem nach Anspruch 3, wobei die Schaltsteuerungs-Schaltungsanordnung die medizinischen Bilddaten vor der ersten Bildverarbeitung zu den mehreren Graphikverarbeitungsschaltungen verteilt, die Ergebnisse der ersten Bildverarbeitung vereinigt und die Vereinigungsdaten an die Speicherschaltungsanordnung ausgibt.

5. Chirurgisches Endoskopsystem nach Anspruch 4, wobei die erste Bildverarbeitung wenigstens einen aus einer Gruppe enthält, die Folgendes umfasst: einen Entwicklungsprozess, einen Detektionsprozess und einen Bildqualitäts-Erhöhungsprozess.

6. Chirurgisches Endoskopsystem nach Anspruch 5, wobei die Zentraleinheit basierend auf den Ergebnissen der ersten Bildverarbeitung Deformationsparameter erzeugt.

7. Chirurgisches Endoskopsystem nach Anspruch 6, wobei bei der zweiten Bildverarbeitung die mehreren Graphikverarbeitungsschaltungen eine Bildverarbeitung basierend auf den Deformationsparametern ausführen.

8. Chirurgisches Endoskopsystem nach Anspruch 7, wobei die Schaltsteuerungs-Schaltungsanordnung ein Bild des vollen Bereichs als die Vereinigungsdaten zu den mehreren Graphikverarbeitungsschaltungen verteilt,
die mehreren Graphikverarbeitungsschaltungen die zweite Bildverarbeitung an einem Zielbereich des vollen Bildes basierend auf den Deformationsparametern ausführen und
wobei der Zielbereich durch die Deformationsparameter bestimmt wird.

9. Chirurgisches Endoskopsystem nach Anspruch 3, wobei die zweite Bildverarbeitung eine affine Transformation für die Bildstabilisierung enthält.

10. Chirurgisches Endoskopsystem nach Anspruch 1, wobei die Ergebnisse von der durch die mehreren Graphikverarbeitungsschaltungen ausgeführten Bildverarbeitung an den Bilddaten, die durch die Schaltsteuerungs-Schaltungsanordnung vereinigt werden, danach zu den mehreren Graphikverarbeitungsschaltungen neu verteilt werden.

11. Chirurgisches Endoskopsystem nach Anspruch 10, wobei die durch die mehreren Graphikverarbeitungsschaltungen nach der Neuverteilung der Bilddaten ausgeführte Verarbeitung unter Verwendung eines Parameters ausgeführt wird, der von der Zentraleinheit empfangen wird und der basierend auf den von den mehreren Graphikverarbeitungsschaltungen vor der Vereinigung zu der Zentraleinheit übertragenen Informationen erzeugt wird.

12. Bildverarbeitungsvorrichtung, die Folgendes umfasst:
eine Schaltsteuerungs-Schaltungsanordnung (312, 322, 323), die konfiguriert ist, eine Verteilung und eine Vereinigung auszuführen;
mehrere Graphikverarbeitungsschaltungen (315), die konfiguriert sind, eine Bildverarbeitung an den über die Verteilung von der Schaltsteuerungs-Schaltungsanordnung empfangenen Bilddaten auszuführen;
eine Zentraleinheit (313), die mit der Schaltschaltungsanordnung und über die Schaltsteuerungs-Schaltungsanordnung mit den mehreren Graphikverarbeitungsschaltungen verbunden ist; und
eine Speicherschaltungsanordnung (314), die durch die Zentraleinheit gemanagt wird,
wobei die Ergebnisse von der durch die mehreren Graphikverarbeitungsschaltungen ausgeführten Bildverarbeitung an den Bilddaten durch die Schaltsteuerungs-Schaltungsanordnung vereinigt werden,
**dadurch gekennzeichnet, dass** die Vereinigung der Ergebnisse unabhängig von der durch die Zentraleinheit gemanagten Speicherschaltungsanordnung ausgeführt wird, bevor die Ergebnisse über die Zentraleinheit an die Speicherschaltungsanordnung ausgegeben werden.

13. Bildverarbeitungsvorrichtung nach Anspruch 11, wobei die durch die mehreren Graphikverarbeitungsschaltungen ausgeführte Bildverarbeitung an den Bilddaten eine unterschiedliche Bildverarbeitung enthält, die durch wenigstens zwei der mehreren Graphikverarbeitungsschaltungen basierend auf einer Anweisung von der Schaltsteuerungs-Schaltungsanordnung ausgeführt wird.

14. Bildverarbeitungsverfahren, das Folgendes umfasst:
Ausführen unter Verwendung mehrerer Graphikverarbeitungsschaltungen (315) einer Bildverarbeitung an medizinischen Bilddaten, die durch ein chirurgisches Endoskop oder Mikroskop erzeugt und über eine Verteilung von einer Schaltsteuerungs-Schaltungsanordnung (312) empfangen werden; und
Vereinigen durch die Schaltsteuerungs-Schaltungsanordnung (312, 322) der Ergebnisse der durch die mehreren Graphikverarbeitungsschaltungen ausgeführten Bildverarbeitung an den Bilddaten,
**dadurch gekennzeichnet, dass** die Vereinigung der Ergebnisse unabhängig von einem durch eine Zentraleinheit (313) gemanagten Speicher (314) ausgeführt wird, bevor die Ergebnisse über die Zentraleinheit an den Speicher ausgegeben werden, wobei die Zentraleinheit mit der Schaltschaltungsanordnung und über die Schaltsteuerungs-Schaltungsanordnung mit den mehreren Graphikverarbeitungsschaltungen verbunden ist.

15. Bildverarbeitungsvorrichtung nach Anspruch 14, wobei die durch die mehreren Graphikverarbeitungsschaltungen ausgeführte Bildverarbeitung an den Bilddaten eine unterschiedliche Bildverarbeitung enthält, die durch wenigstens zwei der mehreren Graphikverarbeitungsschaltungen basierend auf einer Anweisung von der Schaltsteuerungs-Schaltungsanordnung ausgeführt wird.

## Revendications

1. Système d'endoscope chirurgical (10), comprenant :
un endoscope chirurgical (100) configuré pour générer des données d'image médicale ; et
un appareil de traitement d'image, comprenant :
un circuit de commande de commutation (312) recevant les données d'image médicale générées par l'endoscope chirurgical et configuré pour réaliser une distribution et une agrégation ;
une pluralité de circuits de traitement graphique (315) configurés pour réaliser un traitement d'image sur les données d'image médicale reçues par l'intermédiaire de la distribution depuis le circuit de commande de commutation ;
un circuit de traitement central (313) connecté au circuit de commutation et à la pluralité de circuits de traitement graphique par l'intermédiaire du circuit de commande de commutation ; et
un circuit mémoire (314) géré par le circuit de traitement central,
les résultats du traitement d'image sur les données d'image réalisé par la pluralité de circuits de traitement graphique (315) étant agrégés par le circuit de commande de commutation (312, 322),
**caractérisé en ce que** l'agrégation des résultats est indépendante du circuit mémoire (314) géré par le circuit de traitement central (313) avant que les résultats ne soient délivrés au circuit mémoire (314) par l'intermédiaire du circuit de traitement central (313).

2. Système d'endoscope chirurgical selon la revendication 1, le traitement d'image sur les données d'image réalisé par la pluralité de circuits de traitement graphique comprenant un traitement d'image distinct réalisé par au moins deux de la pluralité de circuits de traitement graphique sur la base d'une instruction provenant du circuit de commande de commutation.

3. Système d'endoscope chirurgical selon la revendication 2, la pluralité de circuits de traitement graphique réalisant un premier traitement d'image et un second traitement d'image sur les données d'image médicale,
dans le premier traitement d'image, la pluralité de circuits de traitement graphique réalisant respectivement un traitement d'image sur différentes zones des données d'image médicale.

4. Système d'endoscope chirurgical selon la revendication 3, le circuit de commande de commutation distribuant les données d'image médicale à la pluralité de circuits de traitement graphique avant le premier traitement d'image, agrège les résultats du premier traitement d'image, et délivre les données d'agrégation au circuit mémoire.

5. Système d'endoscope chirurgical selon la revendication 4, le premier traitement d'image comprenant au moins un élément d'un groupe constitué par : un processus de développement, un processus de détection et un processus d'augmentation de la qualité de l'image.

6. Système d'endoscope chirurgical selon la revendication 5, le circuit de traitement central générant des paramètres de déformation sur la base des résultats du premier traitement d'image.

7. Système d'endoscope chirurgical selon la revendication 6, dans le second traitement d'image, la pluralité de circuits de traitement graphique réalisant un traitement d'image sur la base des paramètres de déformation.

8. Système d'endoscope chirurgical selon la revendication 7, le circuit de commande de commutation distribuant une image de zone complète en tant que données d'agrégation à la pluralité de circuits de traitement graphique,
la pluralité de circuits de traitement graphique réalisant le second traitement d'image sur une région cible de l'image complète sur la base des paramètres de déformation, et
la région cible étant déterminée par les paramètres de déformation.

9. Système d'endoscope chirurgical selon la revendication 3, le second traitement d'image comprenant une transformation affine pour la stabilisation de l'image.

10. Système d'endoscope chirurgical selon la revendication 1, les résultats du traitement d'image sur les données d'image réalisées par la pluralité de circuits de traitement graphique qui sont agrégés par le circuit de commande de commutation étant ensuite redistribués à la pluralité de circuits de traitement graphique.

11. Système d'endoscope chirurgical selon la revendication 10, le traitement réalisé par la pluralité de circuits de traitement graphique, après la redistribution des données d'image, étant réalisé en utilisant un paramètre reçu du circuit de traitement central et généré sur la base des informations transmises au circuit de traitement central depuis la pluralité de circuits de traitement graphique avant l'agrégation.

12. Appareil de traitement d'images, comprenant :
un circuit de commande de commutation (312, 322, 323) configurés pour réaliser la distribution et l'agrégation ;
une pluralité de circuits de traitement graphique (315) configurés pour réaliser un traitement d'image sur des données d'image reçues par l'intermédiaire de la distribution depuis le circuit de commande de commutation ;
un circuit de traitement central (313) connecté au circuit de commutation et à la pluralité de circuits de traitement graphique par l'intermédiaire du circuit de commande de commutation ; et
un circuit mémoire (314) géré par le circuit de traitement central,
les résultats du traitement d'image sur les données d'image réalisé par la pluralité de circuits de traitement graphique étant agrégés par le circuit de commande de commutation,
**caractérisé en ce que** l'agrégation des résultats est réalisée indépendamment du circuit mémoire géré par le circuit de traitement central avant que les résultats ne soient délivrés au circuit mémoire par l'intermédiaire du circuit de traitement central.

13. Appareil de traitement d'image selon la revendication 11, le traitement d'image sur les données d'image réalisé par la pluralité de circuits de traitement graphique comprenant un traitement d'image distinct réalisé par au moins deux de la pluralité de circuits de traitement graphique sur la base d'une instruction provenant du circuit de commande de commutation.

14. Procédé de traitement d'image, comprenant :
la réalisation, à l'aide d'une pluralité de circuits de traitement graphique (315), d'un traitement d'image sur des données d'image médicale générées par un endoscope ou un microscope chirurgical et reçues par l'intermédiaire d'une distribution à partir d'un circuit de commande de commutation (312) ; et
l'agrégation, par le circuit de commande de commutation (312, 322), des résultats du traitement d'image sur les données d'image réalisé par la pluralité de circuits de traitement graphique,
**caractérisé en ce que** l'agrégation des résultats est réalisée indépendamment d'une mémoire (314) gérée par un circuit de traitement central (313) avant que les résultats ne soient délivrés à la mémoire par l'intermédiaire du circuit de traitement central, le circuit de traitement central étant connecté au circuit de commutation et à la pluralité de circuits de traitement graphique par l'intermédiaire du circuit de commande de commutation.

15. Appareil de traitement d'image selon la revendication 14, le traitement d'image sur les données d'image réalisé par la pluralité de circuits de traitement graphique comprenant un traitement d'image distinct réalisé par au moins deux de la pluralité de circuits de traitement graphique sur la base d'une instruction provenant du circuit de commande de commutation.
